(19) Europäisches Patentamt
European Patent Office
Office européen des brevets

(11) Publication number: **0 066 415**
**B1**

(12) **EUROPEAN PATENT SPECIFICATION**

(45) Date of publication of patent specification: **02.10.85**

(21) Application number: **82302557.2**

(22) Date of filing: **19.05.82**

(51) Int. Cl.$^4$: **C 07 C 149/437,**
C 07 C 147/00, C 07 C 157/00,
C 07 D 295/12,
C 07 D 307/38, A 61 K 31/17

(54) **N-(arylthioalkyl)-N'-(aminoalkyl)ureas.**

(30) Priority: **20.05.81 US 265278**
**03.02.82 US 345452**

(43) Date of publication of application:
**08.12.82 Bulletin 82/49**

(45) Publication of the grant of the patent:
**02.10.85 Bulletin 85/40**

(84) Designated Contracting States:
**AT BE CH DE FR GB IT LI LU NL SE**

(56) References cited:
**GB-A-1 497 590**
**GB-A-1 604 674**
**US-A-2 832 756**

(73) Proprietor: **A.H. ROBINS COMPANY, INCORPORATED**
**1407 Cummings Drive P.O. Box 26609**
**Richmond Virginia 23261 (US)**

(72) Inventor: **Shanklin, James Robert, Jr.**
**8318 Brookfield Road**
**Richmond Virginia 23227 (US)**
Inventor: **Johnson, Christopher Peter, III**
**3906 West Franklin Street**
**Richmond Virginia 23221 (US)**

(74) Representative: **Kearney, Kevin David Nicholas et al**
**KILBURN & STRODE 30 John Street**
**London, WC1N 2DD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 066 415 B1

**Description**

Field of the Invention

The present invention relates to N-(arylthioalkyl)-N'-(aminoalkyl)ureas and thioureas and oxidation derivatives, acid addition salts and hydrates thereof and a method of treating cardiac arrhythmias therewith and pharmaceutical compositions therefor.

Description of the Prior Art

Oxygen analogs:

N'-[2-(Diethylamino)ethyl]-N-methyl-N-[2-(phenoxy)ethyl]urea, and

N-Methyl-N-[2-(phenoxy)ethyl]-N'-[2-(pyrrolidinyl)ethyl]urea

have been disclosed by Koelzer, P. P. and Wehr, K. H. in Arzneim. Forsch *9,* 113—20 (1959). Anaesthetic activity in animals was disclosed by clinical use was said to be unlikely.

Oxygen analogs in a method of treating cardiac arrhythmias in animals is the subject of copending European Application No. 82 302 556.4 (Publication No. 0066987) claiming priority from U.S. Serial No. 265,510 filed May 20, 1981. The oxidation derivatives, the sulphonyl compounds of the present invention have less central nervous system (CNS) side effects than the corresponding oxygen analogs.

The present invention is concerned with novel N-(arylthioalkyl)-N'-(aminoalkyl)ureas and thioureas and the oxidation derivatives, the sulphinyl and sulphonyl analogs thereof and methods and compositions for treating cardiac arrhythmias in living animals. The compounds have the following structural formula:

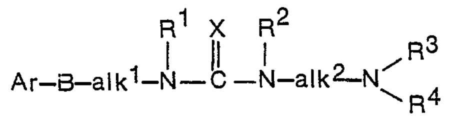

Formula I

wherein;

Ar represents a 1 or 2-naphthyl, 2,3-dihydro-1H-inden-4(or 5)yl, 2-furanyl, phenyl or substituted phenyl group the said substituted phenyl group being substituted by 1—3 radicals which may be the same or different which radicals are selected from loweralkyl, loweralkoxy, halogen, trifluoromethyl, nitro, cyano, or

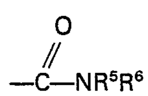

groups, wherein $R^5$ and $R^6$ each represent a hydrogen atom or a loweralkyl group, and Ar may include one intervening methylene group attached to B,

$R^1$ and $R^2$ each represent a hydrogen atom or a loweralkyl, cycloalkyl, phenyl or phenyl-lower alkyl group wherein phenyl may be substituted by a halogen atom or a loweralkyl or loweralkoxy group,

X represents an oxygen or sulphur atom,

B represents a thio, sulphinyl or sulphonyl group,

$R^3$ and $R^4$ each represent a hydrogen atom or a lower-alkyl, phenyl or phenyl-loweralkyl group wherein phenyl may be substituted by a halogen atom or a loweralkyl or loweralkoxy group, and may be the same or different, or $R^3$ and $R^4$ taken together with the adjacent nitrogen may form a heterocyclic residue,

$alk^1$ and $alk^2$ each represent a loweralkylene or loweralkylene-loweralkyl group and may be the same or different,

and the pharmaceutically acceptable addition salts and hydrates thereof.

In the further definition of symbols in the formulas hereof and where they appear elsewhere throughout this specification and claims, the terms have the following significance.

The term "loweralkyl" as used herein means straight and branched chain radicals of up to eight carbons inclusive and is exemplified by such groups as methyl, ethyl, propyl, isopropyl, butyl, sec. butyl, tert-butyl, amyl, isoamyl, hexyl, heptyl, and octyl radicals. The term "loweralkoxy" has the formula —O—loweralkyl.

The term "cycloalkyl" as used herein means primarily cyclic alkyl radicals containing 3 to 9 carbon atoms inclusive and includes such groups as cyclopropyl, cyclobutyl, cyclopentyl, cyclohexyl, methylcyclohexyl, and cycloheptyl.

The term "halogen" when referred to herein means fluorine, chlorine, bromine and iodine, preferably fluorine, chlorine and bromine.

The term "loweralkylene" as used herein means connecting hydrocarbon groups represented by methylene ($—CH_2—$) ethylene ($—CH_2—CH_2—$), and propylene ($—CH_2—CH_2CH_2—$). The term "loweralkylene-loweralkyl" means the hydrocarbon groups ethylidine

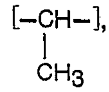, 1,2-propylene 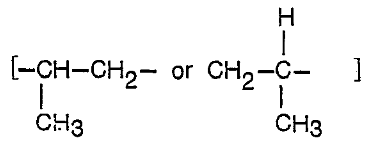,

isopropylidene 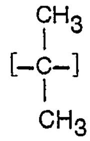 and 1,3-butylene 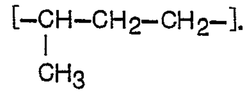.

The term "heterocyclic residue" as used herein means pyrrolidine, piperidine, piperazine, 4-loweralkylpiperazine or morpholino radicals.

"Pharmaceutically acceptable acid addition salts" are those salts formed by the N-(arylthioalkyl)-N'-(aminoalkyl)ureas and thioureas and oxidation derivatives of this invention with any acid which is physiologically compatible in warm blooded animals, such salts being formed by either strong or weak acids. Representative of strong acids are hydrochloric, sulphuric and phosphoric acids. Representative of weak acids are fumaric, maleic, succinic, oxalic, citric, tartaric and cyclohexamic acids.

The compounds of the present invention exhibit antiarrhythmic activity in dogs, several arrhythmia models in which arrhythmia is induced by one or more of the following as described more fully hereinbelow under pharmacology: (1) Ouabain, (2) Ligation, (3) Injury, and (4) Acetylcholine.

It is therefore an object of the present invention to provide novel N-(arylthioalkyl)-N'-(aminoalkyl)ureas and thioureas and oxidation derivatives thereof which have a high degree of cardiac activity in animals.

Another object of the invention is to provide compounds for use in treatment of living animals for the purpose of alleviating cardiac arrhythmias and compositions therefor.

Detailed Description of the Invention

The invention comprises N-(arylthioalkyl)-N'-(aminoalkyl)ureas and thioureas and derivatives thereof as set forth hereinabove in Formula I for use in treating arrhythmias in living animals. The invention also extends to pharmaceutical compositions comprising compounds set out above as Formula I which may be administered to a living animal body for cardiac arrhythmic effect in an amount effective to control arrhythmia.

The present invention may be put into practice in various ways and a number of specific embodiments will be described to illustrate the invention. A general discussion of preparative methods will be given first followed by reference to preparations 1 to 49 which are of starting materials and Examples 1—60 which are of compounds in accordance with the invention and Examples 61 to 65 which are of compositions in accordance with the invention.

The compounds of formula I may be prepared by a choice of four methods, A, B, C or D as follows:

*Method A.* This method is represented by the following equation:

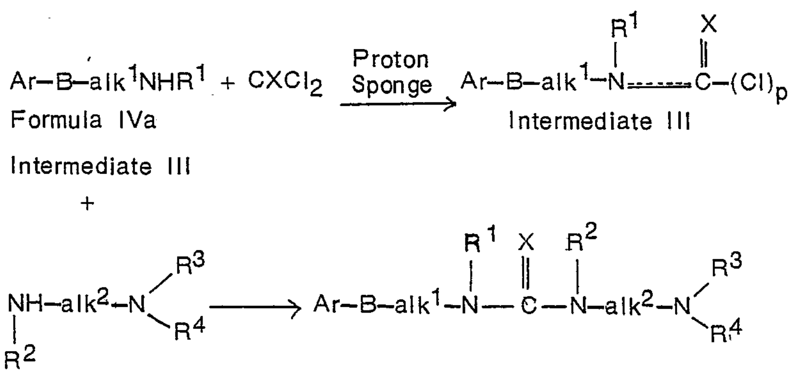


Formula IVa — Intermediate III — Intermediate III — Formula IIa — Formula I

wherein Ar, $alk^1$, $alk^2$, $R^1$, $R^2$, X, $R^3$ and $R^4$ are as defined above and B represents a sulphur atom or a

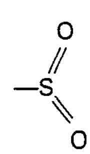

group. When $R^1$ represents hydrogen, p is zero and the dotted line is a double bond forming an isocyanate;

otherwise, when p is 1, the dotted line has no significance, with the proviso that when $R^2$ is not H, $R^3$ and $R^4$ must be other than hydrogen or $R^2$ is the same as $R^3$, and $R^4$ is hydrogen.

Generally in Method A, the Formula IV is a compound reacted with phosgene (or thiophosgene) in a suitable organic solvent plus Proton Sponge (U.S. Registered Trade Mark) which is the compound, 1,8-bis(dimethylamino)naphthalene, followed by extraction (washing) with dilute sulphuric acid and the organic layer is dried and evaporated to an oil residue (Intermediate Formula III) which may be isolated if desired. The oil is dissolved in a solvent such as tetrahydrofuran and reacted with an amine of Formula IIa. The reaction mixture is stripped of solvents (to dryness) and the residue partitioned between water and a solvent such as chloroform. Evaporation of the solvent yields an oil which may or may not crystallize. Pharmaceutically acceptable salts may be prepared by reacting with an appropriate acid. The method is illustrated more specifically in Example 1 and other examples utilizing phosgene or thiophosgene.

*Method B.* This method is represented by the following equation:

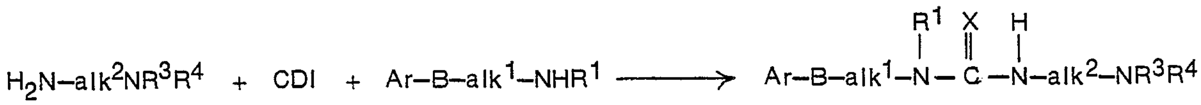

Formula IIb Formula IVa Formula Ib

where CDI is 1,1′-carbonyldiimidazole
or 1,1′-thiocarbonyldiimidazole
wherein Ar, B, $alk^1$, $alk^2$, $R^1$, $R^3$, $R^4$ and X are as defined hereinabove under Formula I above, except $R^3$ and $R^4$ cannot be H. Formula Ib is encompassed by Formula I and $R^2$ is always hydrogen in this method.

Generally in Method B, the formula IIb compound is reacted first with 1,1′-carbonyldiimidazole in a suitable solvent (e.g., tetrahydrofuran) followed by reaction with a solution of an arylthio, arylsulphinyl or arylsulphonyl alkylamine. The reaction mixture is quenched in water and extracted with a suitable solvent (e.g., methylene chloride) or the reaction mixture is evaporated to dryness and the residue partitioned between water and a suitable solvent. In either case the organic layer is dried and evaporated to yield an oil, the free base. Pharmaceutically acceptable acid addition salts may then be prepared with a suitable acid. The method is illustrated more fully in Example 7.

*Method C.* This method is represented by the following equation:

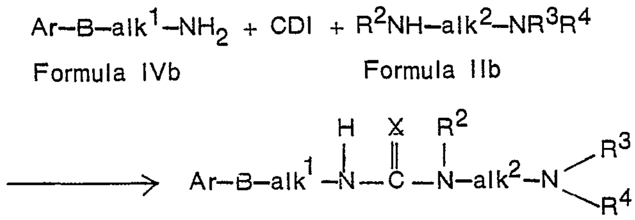

Formula IVb Formula IIb

Formula Ic

where CDI is 1,1-carbonyldiimidazole or 1,1′-thiocarbonyldiimidazole
wherein Ar, B, $R^2$, $R^3$, $R^4$ and $alk^2$ are as defined hereinabove, and $R^1$ is always hydrogen in this method. Formula Ic is encompassed by Formula I.

Generally in Method C, the formula IVb compound is reacted first with 1,1′-carbonyldiimidazole or thiocarbonyldiimidazole in a suitable solvent (e.g., tetrahydrofuran) followed by reaction with an alkyldiamine having one free hydrogen. The solvent is removed by evaporation and the residue partitioned between a suitable solvent (e.g., chloroform) and water. The free base is obtained by evaporation and may be converted to a pharmaceutically acceptable salt with a suitable acid. The method is illustrated more fully in Example 32.

*Method D.* This method is represented by the following equation:

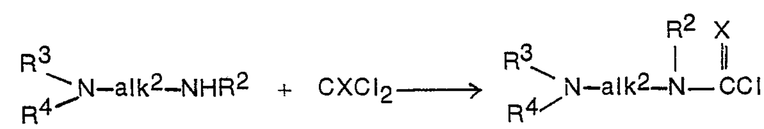

Formula VI Intermediate V

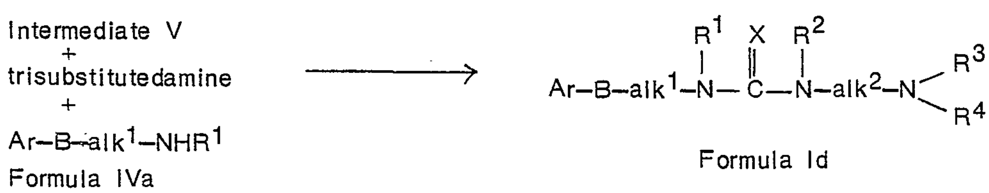


Formula IVa Formula Id

wherein Ar, $alk^1$, $alk^2$, B, $R^1$, $R^2$, $R^3$ and $R^4$ have the values assigned above, except $R^2$, $R^3$ and $R^4$ are never hydrogen. Formula Id is encompassed by Formula I.

Generally in Method D, illustrated more fully in Example 33, a trisubstituted diaminoalkyl (Formula VI) and phosgene, in a suitable solvent (e.g., methylene chloride) are reacted to give an intermediate product (Formula V) which may be isolated if desired, which is then reacted with Ar-B-$alk^1$-$NHR^1$ (primary or secondary amine (Formula IVa) and trisubstituted amine e.g. triethylamine. The product is isolated by conventional extraction and evaporation methods.

Starting compounds of Formula IVa wherein B represents a sulphur atom may be prepared by reacting aryl-S-$alk^1$-halides with the appropriate amine, and compounds of Formula IVa wherein B represents a

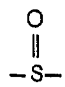 or 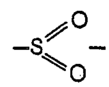

group obtained by further reaction with sodium perborate. The equations are:

$$(1)\quad Ar{-}S{-}alk^1{-}halo + \overset{Excess}{NH_2R^1} \xrightarrow[pressure]{heat} Ar{-}S{-}alk^1NHR^1$$

Formula IVa–1

(2) 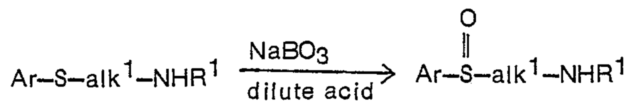


Formula IVa–2

(3) 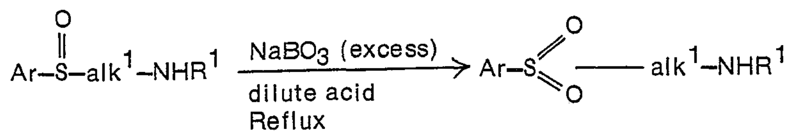


Formula IVa–3

Starting compounds of Formula IVa wherein B represents an

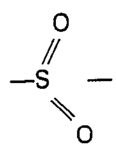

group may also be prepared by reacting arythioalkylamines with phenyl chloroformate followed by oxidation with m-chloroperoxybenzoic acid and hydrolysis with hydrobromic acid. The equations are:

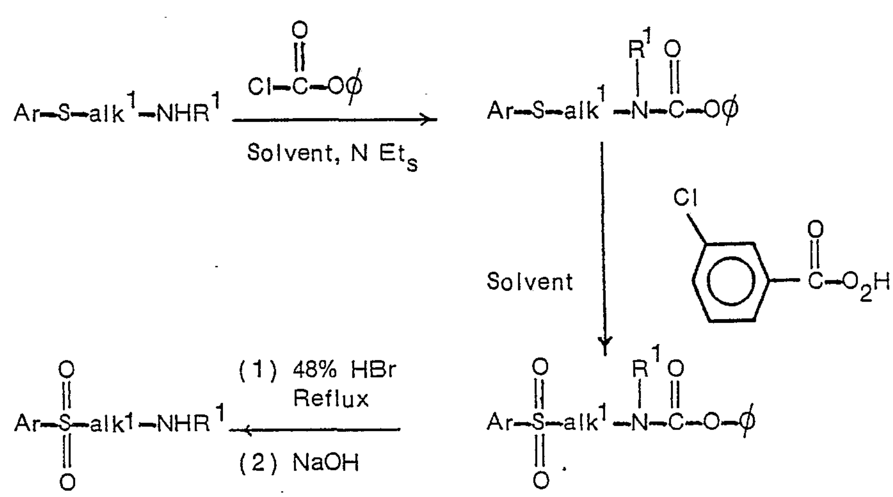

Alternatively, starting compounds of Formula IVa wherein B represents a sulphur atom or an

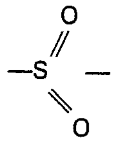

group may be prepared via mesyl derivatives as illustrated by the following equations:

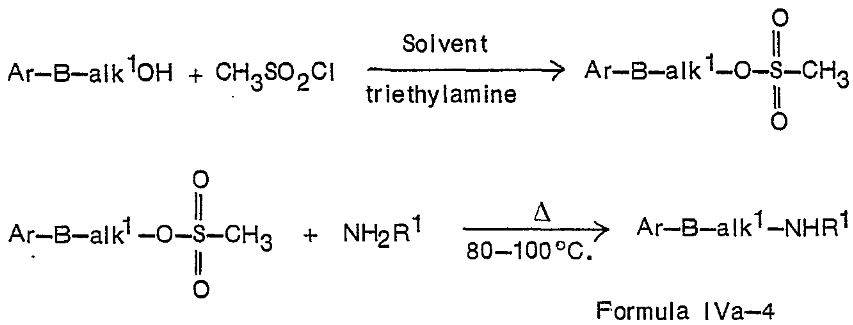


Formula IVa–4

wherein Ar, alk[1] and R[1] are as defined under Formula I above. Reaction (1) may be carried out by heating the reactants in a closed container such as a bomb. Reaction (2) may be carried out at room temperature and Reaction (3) may be conducted at reflux. Reaction (2) as a step may be omitted by going directly to excess sodium perborate at reflux temperature.

Some of the Ar-B-alk[1]-halide starting materials are available commercially. Some of the halides wherein B represents a sulphur atom may be prepared from metal salts of arylsulphides and α,ω-dihaloalk[1] compounds in refluxing ethanol as represented by the following equation:

$$ArS^-M^+ + Br{-}alk^1{-}halo \xrightarrow[EtOH]{Reflux} Ar{-}S{-}alk^1{-}halo$$

Alternately, the chloro starting analogs may be prepared from arylsulphide and α-chloro-ω-hydroxy alkanes followed by reaction with thionyl chloride as represented by the following equation:

$$Ar{-}S^-M^+ + Cl{-}alk^1{-}OH \xrightarrow[EtOH]{Reflux} Ar{-}S{-}alk^1{-}OH$$

$$Ar{-}S{-}alk^1{-}OH + SOCl_2 \longrightarrow Ar{-}S{-}alk^1{-}Cl$$

The sulphinyl and sulphonyl starting analogues are obtained by oxidation with hydrogen peroxide in acetic acid as follows:

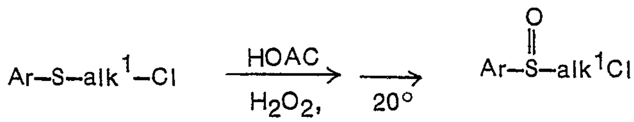


and

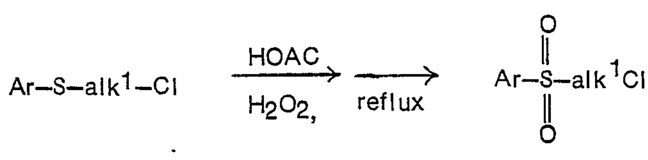

The precursor aryl sulphides may be purchased or may be prepared as illustrated by the following equation:

Method of Org. Synth. *51*, 139—142

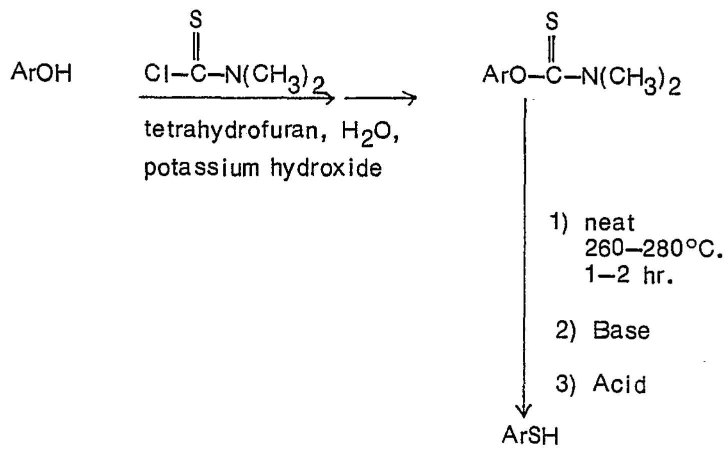


Starting compounds of Formula IVb are prepared as illustrated by the following equation:

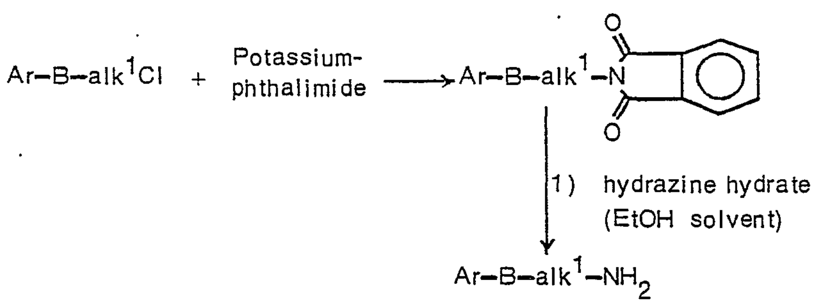


The starting compounds of Formula IIb and VI either may be purchased or may be prepared by usual means.

Preparations 1—39 and 44—49 provide intermediates of Formula IV, or are used in the preparation thereof, which compounds have the composite formula:

$$Ar—B—alk^1NR^1R^2 \qquad \text{(Formula IV)}$$

which encompasses Formulas IVa and IVb.

Preparations 40—43 illustrate the preparation of compounds of Formula IIb having a phenyl-loweralkyl moiety.

Preparation 1

N-[2-[(4-Chlorophenyl)thio]ethyl]-1-methylethanamine hydrochloride

A solution of 24.0 g (0.116 mole) chloroethyl-p-chlorophenyl sulphide in 100 ml of isopropylamine was agitated overnight in a stainless steel bomb at 80°C. The reaction mixture was then stripped to dryness and the residue partitioned between water and chloroform. The chloroform layer was extracted with 1 N sulphuric acid. Three layers were obtained: a water phase (lower), a chloroform phase (upper) and an intermediate phase. The aqueous and intermediate phases were combined, made alkaline and extracted with chloroform. Evaporation yielded an oil, the free base of the title compound. A portion of the oil was reacted with ethereal hydrogen chloride and the resulting hydrochloride salt was recrystallized from methanol-diethyl ether to give 2.23 g (22.6%) of white crystalline product, m.p. 126—128°C.

Analysis:

Calculated for $C_{11}H_{17}NSCl_2$: C, 49.63; H, 6.44; N, 5.26

Found: C, 49.78; H, 6.50; N, 5.50

Preparation 2

1-Methyl-N-[2-(2-naphthalenylthio)ethyl]ethanamine, hydrochloride.

2-Naphthalenethiol was prepared by the method of Org. Syn. *51*, pp. 139—142.

A solution of the potassium salt of 2-naphthalenethiol was prepared by reacting 50.89 g (0.32 mole) of 2-naphthalenethiol and 17.92 g (0.32 mole) of potassium hydroxide in 500 ml of ethanol. To the solution was added 297.6 g (1.6 mole) of 1,2-dibromoethane. The solution was refluxed overnight, stripped to

dryness and the residue dissolved in chloroform. The chloroform layer was extracted with water and 10% sodium hydroxide. The chloroform layer was evaporated to leave a dark-brown oil as residue which contained about 20% of an unwanted dimer, 1,2-bis naphthalenylthioethane. The impure mixture was stirred overnight with 100 ml of isopropylamine. The reaction mixture was evaporated to dryness and the residue partitioned between chloroform and water. Evaporation of the chloroform layer gave an oil. The oil was triturated with methanol and chilled and a precipitate was filtered off which proved to be the dimer: 1,2-bis-(2-thionaphthylene) ethane. The filtrate was treated with ethereal hydrogen chloride to give the title salt as white crystals weighing 10.2 g (11.3%), m.p. 137.5—138.5°C.

Analysis:

Calculated for $C_{15}H_{20}NSCl$: C, 63.92; H, 7.15; N, 4.97
Found: C, 63.99; H, 7.20; N, 5.10

Preparation 3

N-[2-[(4-Chlorophenyl)sulphinyl]ethyl]-1-methylethanamine

A solution of 15.05 g (0.066 mole) of N-[2-[(4-chlorophenyl)thio]ethyl]-1-methylethanamine and 12.0 g (0.0779 mole) of sodium perborate in 400 ml of 1 M sulphuric acid was stirred at room temperature for about 18 hr. The solution was made basic with 50% sodium hydroxide and the basic solution was extracted with methylene chloride. The methylene chloride layer was dried with magnesium sulphate and the solvent removed *in vacuo* to give 14.7 g of solid. Nuclear magnetic resonance analysis showed a 9 to 1 ratio of sulphoxide to sulphide. The solid was recrystallized from ether-hexane to give 12.3 g (76.4%) of the free base title compound as crystalline solid, m.p. 52.5—53.5°C.

Preparation 4

N-[2-[(4-Chlorophenyl)sulphinyl]ethyl]-1-methylethanamine, maleate [1:1]

A portion of the free base obtained in Preparation 3 was reacted with maleic acid to give the maleate salt which was recrystallized from methanol-diethyl ether to give a white crystalline solid, m.p. 158.5—159°C.

Analysis:

Calculated for $C_{15}H_{20}NO_5SCl$: C, 49.79; H, 5.57; N, 3.87
Found: C, 49.91; H, 5.58; N, 3.89

Preparation 5

N-[2-[(4-Chlorophenyl)sulphonyl]ethyl]-1-methylethanamine, hydrochloride

A solution of 51.51 g (0.216 mole) of 2-chloroethyl-p-chlorophenyl sulphone in 400 ml of isopropylamine was stirred at room temperature for about 72 hr. The isopropylamine was removed *in vacuo* and the residue was dissolved with agitation in a mixture of methylene chloride and dilute sodium hydroxide. The methylene chloride layer was extracted several times with dilute sodium hydroxide and dried over magnesium sulphate. The solvent was removed *in vacuo* to give 53.5 g oil, the free base of the title compound. A portion of the oil was converted to the hydrochloride salt which was recrystallized from methanol-diethyl ether to give a white crystalline solid, m.p. 169—170°C.

Analysis:

Calculated for $C_{11}H_{17}NO_2SCl_2$: C, 44.30; H, 5.75; N, 4.70
Found: C, 44.37; H, 5.81; N, 4.73

Preparation 6

1-Methyl-N-[2-(phenylsulphonyl)ethyl]ethanamine hydrochloride

A solution of 49.63 g (0.24 mole) of 2-chloroethylphenyl sulphone in 300 ml of isopropylamine was stirred at room temperature for about 20 hr. The isopropylamine was removed *in vacuo* and the residue was dissolved with agitation in a mixture of methylene chloride and dilute sodium hydroxide. The methylene chloride layer was extracted several times with dilute sodium hydroxide and dried over magnesium sulphate. The solvent was removed *in vacuo* to give 55.1 g of oil, the free base of the title compound. A portion of the oil was converted to the hydrochloride salt with ethereal hydrogen chloride which was recrystallized from methanol-diethyl ether to give white crystals, m.p. 151—152.5°C.

Analysis:

Calculated for $C_{11}H_{18}NO_2SCl$: C, 50.09; H, 6.88; N, 5.31
Found: C, 50.12; H, 6.91; N, 5.31

Preparation 7

1-[2-[(4-Chlorophenyl)sulphonyl]ethyl]-1H-isoindole-1,3(2H)dione

A mixture of 14.22 g (0.059 mole) of 2-chloroethyl-p-chlorophenylsulphone and 11.60 (0.06 mole) of potassium phthalimide in 200 ml of dimethylformamide was stirred at 80°C for 2 hr. The reaction mixture was quenched in water and the white precipitate which resulted was collected by filtration. Recrystallization from diethyl ether gave a white solid, m.p. 154—155.5°C.

Analysis:

Calculated for $C_{16}H_{12}NO_4SCl$: C, 54.94; H, 3.46; N, 4.00
Found: C, 54.60; H, 3.41; N, 3.90

Preparation 8

2-[(4-Chlorophenyl)sulphonyl]ethanamine, monohydrochloride

A solution of 1-[2-(4-chlorophenyl)sulphonyl]ethyl]-1H-isoindole-1,3(2H)dione and 85% hydrazine in 95% ethanol was refluxed for 2 hr. The reaction mixture was quenched in dilute sulphuric acid and the white solid which precipitated was filtered off. The aqueous solution was made basic with sodium hydroxide and extracted with methylene chloride. The methylene chloride layer was dried over magnesium sulphate and the solvent evaporated to give an oil as residue. The oil was dissolved in methanol and the solution treated with an excess of ethereal hydrogen chloride. The product precipitated as a white solid, m.p. 218.5—219.5°C.

Analysis:

Calculated for $C_8H_{11}NO_2SCl_2$: C, 37.51; H, 4.33; N, 5.47
Found: C, 37.52; H, 4.34; N, 5.59

Preparation 9

2-[3-[(4-Chlorophenyl)sulphonyl]propyl]-1,3-dihydro-2H-isoindole-1,3-dione

A solution of 17.8 g (0.07 mole) of 1-chloro-3-(p-chlorophenylsulphonyl)propane and 15.2 g (0.082 mole) of potassium phthalimide in 300 ml of dimethylformamide was stirred at 90—110°C. for 2 hr and then quenched in water. The mixture was extracted with methylene chloride and the methylene chloride layer dried over magnesium sulphate. Diethylether and hexane were added and the solution stored at 0°C overnight. The crystalline product was recrystallized from methylene chloride diethyl ether to give a white crystalline product, m.p. 187.5—188.5°C.

Analysis:

Calculated for $C_{17}H_{14}NO_4SCl$: C, 56.12; H, 3.88; N, 3.85
Found: C, 56.10; H, 3.94; N, 3.87

Preparation 10

3-[(4-Chlorophenyl)sulphonyl]-1-propanamine, fumarate [2:1]

A mixture of 55.92 g (0.154 mole) of 2-[3-[(4-chlorophenyl)sulphonyl]propyl]-1,3-dihydro-2H-isoindole-1,3-dione and 11.8 g (0.2 mole) hydrazine hydrate, 85%, was refluxed for 4 hr in 750 ml of 95% ethanol. The reaction mixture was poured into ice, diluted to 3 litres and made alkaline with 10% sodium hydroxide. The diluted mixture was extracted with methylene chloride and the organic phase was extracted with 1 N sulphuric acid. The acidic layer was made alkaline and extracted with methylene chloride. Evaporation of the methylene chloride gave an oil which crystallized to a white solid, the free base of the title compound. The oil was reacted with fumaric acid and the salt recrystallized from methanol, yielding 2.95 g (41%) white crystalline product, m.p. 232—234°C.

Analysis:

Calculated for $C_{22}H_{28}N_2O_8S_2Cl_2$: C, 45.29; H, 4.84; N, 4.80
Found: C, 45.45; H, 4.83; N, 4.85

Preparation 11

1-Methyl-N-[2-(1-naphthalenylthio)ethyl]ethanamine, hydrochloride

To a solution of 143.7 g (0.898 mole) of 1-naphthalenethiol and 50.3 g (0.898 mole) potassium hydroxide in 1 liter of 95% ethanol, which had been stirred for 10 minutes at room temperature, was added 1,843.5 g (4.49 moles) of dibromethane. The resulting solution was heated overnight at gentle reflux, filtered and stripped to dryness. The residue was dissolved in chloroform and extracted with 10% sodium hydroxide. The chloroform layer was evaporated leaving a dark brown oil as residue. The oil was placed in a bomb with 200 ml of isopropyl amine and agitated overnight at 100°C. The mixture was evaporated to give an oil which was extracted with water and 10% aqueous sodium hydroxide solution. The chloroform layer was then extracted with 1 N sulphuric acid solution. The acidic layer was made alkaline with 50% sodium hydroxide and extracted with chloroform. Evaporation of the chloroform layer gave a dark brown oil, the free base of the title compound. The oil was reacted with ethereal hydrogen chloride and the salt recrystallized from methanol-diethyl ether to give 44.3 g (17.3%) of white crystalline product, m.p. 121—122.5°C.

Analysis:

Calculated for $C_{15}H_{20}NSCl$: C, 63.92; H, 7.15: N, 4.97
Found: C, 63.67; H, 7.17; N, 4.87

Preparation 12

1-Methyl-N-[2-[(1-naphthalenyl)sulphonyl]ethyl]ethanamine, hydrochloride

To a solution of 12.0 g (0.0426 mole) of 1-methyl-N-[2-(1-naphthalenylthio)ethyl]ethanamine (last oil in Preparation 11) in 500 ml of 2N $H_2SO_4$ was added 18.4 g (0.12 mole) of sodium perborate tetrahydrate. The mixture was heated overnight at reflux after which time it was determined that approximately half of the starting material had been converted to the sulphoxide and the other half to the sulphone. The mixture was treated with 30.8 g (0.2 mole) more sodium perborate in acid and refluxed. The reaction mixture was cooled with ice and made alkaline with 50% sodium hydroxide and extracted with chloroform. Evaporation of the

chloroform layer gave 8.60 g dark-brown oil, the free base of the title compound. A 3 g sample was reacted with ethereal hydrogen chloride and the salt recrystallized from methanol-diethyl ether to give 2.83 g (16.8% yield) of white crystalline solid, m.p. 165—167°C.

Analysis:

Calculated for $C_{15}H_{20}NO_2SCl$: C, 57.41; H, 6.42; N, 4.46

Found: C, 57.33; H, 6.42; N, 4.50

Preparation 13

1-Methyl-N-[2-[(4-methylphenyl)thio]ethyl]ethanamine, hydrochloride

A solution of 10 g (0.035 mole) of 1-p-thiocresyl-2-methane sulphonyl ethane in 50 ml of isopropyl amine was heated at 100°C overnight in a bomb. The reaction mixture was cooled to room temperature and stripped to dryness. The resulting oil residue was dissolved in chloroform and the solution extracted with 1 N sulphuric acid. The acidic layer was carefully made basic with 50% aqueous sodium hydroxide and extracted with chloroform. Evaporation of the chloroform gave an oil, the free base of the title compound. The oil was reacted with ethereal hydrogen chloride and the salt obtained was recrystallized from methanol-diethyl ether to give 4.5 g (53.1%) of white crystalline solid, m.p. 146—147°C.

Analysis:

Calculated for $C_{12}H_{20}SNCl$: C, 58.64; H, 8.20; N, 5.70

Found: C, 58.64; H, 8.29; N, 5.71

Preparation 14

1-Methyl-N-[2-[(4-methylphenyl)sulphonyl]ethyl]ethanamine, hydrochloride [1:1]

A solution of 7.61 g (0.0346 mole) of 1-methyl-N-[2-[(4-methylphenyl)thio]ethyl]ethanamine (obtained as oil in Preparation 13) and 30.3 g (0.2 mole) of sodium perborate, tetrahydrate in 500 ml of 2 N sulphuric acid was refluxed overnight. The reaction mixture was cooled and made alkaline with 50% sodium hydroxide ice mixture and extracted with chloroform. Evaporation of the chloroform layer gave an oil residue, the free base of the title compound. A portion of the oil was reacted with ethereal hydrogen chloride. The salt obtained was recrystallized from methanol-diethyl ether to give a white crystalline product, m.p. 188—190°C in 62.5% yield.

Analysis:

Calculated for $C_{12}H_{20}NO_2SCl$: C, 51.88; H, 7.26; N, 5.04

Found: C, 51.24; H, 7.20; N, 5.02

Preparation 15

N-[2-(2,3-Dihydro-1H-inden-4-yl)sulphonyl]-1-methylethanamine, and
N-[2-(2,3-Dihydro-1H-inden-5-yl-sulphonyl]-1-methylethanamine

2-(2,3-Dihydro-1-H-indene-4-yl-thiol and 2-(2,3-dihydro-1-H-indene-5-yl thiol were first prepared by the method of Org. Syn. *51*, pp. 139—142 used for the preparation of 2-naphthalenethiol.

From these thiols were prepared:

1-Methyl-N-[2-(4-indanethio)ethyl]ethanamine and 1-methyl-N-[2-(5-indanethio)ethyl]ethanamine, utilizing the method of Preparation 2. The title compounds were prepared therefrom by hot oxidation with sodium perborate as in Preparation 12.

Preparations 16A to 16E

Following the procedure of Preparation 5, substituting the following for 2-chloroethyl-p-chlorophenyl sulphone:

2-chloroethyl 3,5-dichlorophenylsulphone,
2-chloroethyl 3,4,5-trimethoxyphenylsulphone,
2-chloroethyl 4-trifluoromethylphenylsulphone,
2-chloroethyl 4-cyanophenylsulphone,
2-chloroethyl 4-nitrophenylsulphone,

there were obtained:

N-[2-[(3,5-dichlorophenyl)sulphonyl]ethyl]-1-methylethanamine, (Preparation 16A)
N-[2-[(3,4,5-trimethoxyphenyl)sulphonyl]ethyl]-1-methylethanamine, (Preparation 16B)
1-methyl-N-[2-[4-trifluoromethylphenyl)sulphonyl]ethyl]ethanamine, (Preparation 16C)
N-[2-[(4-cyanophenyl)sulphonyl]ethyl-1-methylethanamine, (Preparation 16D) and
1-methyl-N-[2-[(4-nitrophenyl)sulphonyl]ethyl]ethanamine (Preparation 16E).

Preparations 17A to 17C

Following the procedure of Preparation 6 but substituting the following amines for isopropylamine:

cyclohexylamine,
aniline, and
benzylamine

there were obtained:

N-cyclohexyl-N-[2-(phenylsulphonyl)ethyl]amine, (Prep. 17A)
N-phenyl-N-[2-(phenylsulphonyl)ethyl]amine, (Prep. 17B) and
N-benzyl-N-[2-(phenylsulphonyl)ethyl]amine (Prep. 17C).

Preparation 18

1-Methyl-N-[2-(1-naphthalenesulphinyl)ethyl]ethanamine, hydrochloride

A solution of 12.0 g (0.043 mole) of 1-methyl-N-[2-(1-naphthalenylthio)ethyl]ethanamine hydrochloride and 19.7 g (0.028 mole) of sodium perborate tetrahydrate in 500 ml of 2 N sulphuric acid was stirred overnight at room temperature. The solution was poured over ice and the mixture was made alkaline with 50% sodium hydroxide and then extracted with chloroform. The chloroform layer was evaporated to give a dark-brown oil, the free base of the title compound, which crystallized at room temperature. A one gram sample was reacted with ethereal hydrogen chloride and recrystallized from methanol-diethyl ether to give 0.72 g (9.1%) of white solid, m.p. 153—155°C.

Analysis:

Calculated for $C_{15}H_{20}NSOCl$: C, 60.49; H, 6.77; N, 4.70
Found: C, 60.17; H, 6.75; N, 4.75

Preparation 19

1-Methyl-N-[2-(4-methylphenyl)sulphinyl]ethyl]ethanamine, hydrochloride

A solution of 12.3 g (0.05 mole) of 1-methyl-N-[2-[(4-methylphenyl)thio]ethyl]ethanamine (oil in Preparation 13) in 500 ml of 2 N sulphuric acid was stirred overnight at room temperature with 23.1 g (0.15 mole) of sodium perborate tetrahydrate. The reaction mixture was made alkaline and extracted with chloroform. Evaporation to remove chloroform gave an oil, the free base of the title compound (11.7 g). A portion of the oil was converted to the hydrochloride, a white salt, (ca. 100% yield), m.p. 127—128°C., by reacting with ethereal hydrogen chloride.

Analysis:

Calculated for $C_{12}H_{20}NSOCl$: C, 55.05; H, 7.70; N, 5.35
Found: C, 54.51; H, 7.68; N, 5.41

Preparation 20

N-[2-[(2,3-Dihydro-1H-inden-4-yl)thio]ethyl]-2-propanamine, hydrochloride

A solution of 42.27 g (0.73 mole) of 1-o-mesyl-2 (4-thioindane)ethane (also known as purple oil −50%) in 200 ml of isopropylamine was heated at 100°C overnight in a bomb. The reaction mixture was cooled to room temperature and stripped to dryness. The residue was dissolved in chloroform and extracted with 1 N sulphuric acid. The acidic layer was made alkaline and extracted with chloroform. Removal of solvent from the last chloroform layer gave a dark-brown oil. The oil was dissolved in methanol and refrigerated overnight. The solution was filtered to remove a white solid. The solution was filtered to remove a white solid. The filtrate was treated with ethereal hydrogen chloride and refrigerated. A solid was obtained on filtering which, after drying overnight *in vacuo,* was light brown in color, m.p. 196—197°C

Analysis:

Calculated for $C_{14}H_{22}NSCl$: C, 61.86; H, 8.16; N, 5.15
Found: C, 62.02; H, 8.19; N, 5.28

Preparation 21

Methanesulphonic Acid [2-[(3,4-dichlorophenyl)thio]ethyl]ester

To a solution of 117.97 g (0.53 mole) of 2-[(3,4-dichlorophenyl)thio]ethanol (prepared by reacting 3,4-dichlorothiophenol and 2-chloroethanol) and 53.5 g (0.53 mole) of triethylamine in 500 ml of benzene was added dropwise a benzene solution of 60.9 g (0.53 mole) of methane sulphonyl chloride over a one hour period with cooling in an ice bath. The reaction mixture was stirred at room temperature overnight and filtered. Solvent was removed from the filtrate in a rotary evaporator to give an oil which crystallized. A portion was recrystallized from isopropyl ether to give white crystalline solid, m.p. 53—55°C.

Analysis:

Calculated for $C_9H_{10}S_2O_3Cl_2$: C, 35.89; H, 3.35;
Found: C, 35.81; H, 3.43

Preparation 22

N-[2-[(3,4-Dichlorophenyl)thio]ethyl]-2-propanamine, hydrochloride

A solution of 151.46 g (0.548 mole) of methanesulphonic acid [2-[(3,4-dichlorophenyl)thio]ethyl]ester in 100 ml of isopropylamine was heated overnight in a bomb at 100°C. The isopropylamine was removed in a rotary evaporator and the residue partitioned between chloroform and 5% sodium hydroxide. The chloroform was removed by rotary evaporator to give an oil, the free base of the title compound. The oil was dissolved in methanol and converted to the hydrochloride salt with ethereal hydrogen chloride. Recrystallization of the salt from methanol-diethyl ether gave 101.53 g (61.6%) of white crystalline product, m.p. 132—133.5°C.

Analysis:

Calculated for $C_{11}H_{16}NSCl_3$: C, 43.94; H, 5.36; N, 4.66
Found: C, 44.09; H, 5.34; N, 4.79

Preparation 23

N,N-Dimethylcarbamothioic Acid -O-(2,3-dihydro-1H-inden-4-yl)ester

A solution of the potassium salt of 4-indanol was prepared by dissolving 20.12 g (0.15 mole) of 4-indanol in 100 ml of water containing 8.40 g (0.15 mole) of potassium hydroxide. The solution was cooled to 0°C using an ice-salt bath and a solution of 24.8 g (0.2 mole) of dimethylthiocarbamyl chloride in 100 ml of tetrahydrofuran was added dropwise with stirring while keeping the temperature at 0—5°C. The bath was removed and the reaction mixture stirred for 20—30 minutes. The reaction mixture was made alkaline by adding 50 ml of 10% potassium hydroxide and then extracted with benzene. The benzene layer was then back-extracted with a saturated sodium chloride solution. On evaporation to remove solvent a dark-brown oil residue was obtained which on crystallizing from methanol gave 16.0 g (48.3%) of white crystalline product, m.p. 74—76°C.

Analysis:

Calculated for $C_{12}H_{15}NOS$: C, 65.12; H, 6.83; N, 6.33

Found: C, 64.76; H, 6.80; N, 6.38

Preparation 24

2-[3-(Phenylthio)propyl)-1H-isoindole-1,3-(2H)dione

A solution of 32.86 g (0.177 mole) of 1-chloro-3-(phenylthio)propane and 33.7 g (0.182 mole) of potassium phthalimide in 500 ml of dimethylformamide was stirred at 80°C for 19 hr. The dimethylformamide was removed *in vacuo*. The residue was dissolved in methylene chloride and the resulting solution extracted with several portions of dilute sodium hydroxide solution. The methylene chloride layer was dried over magnesium sulphate, filtered, and the filtrate evaporated *in vacuo*. The resulting solid residue was recrystallized from methylene chloride-hexane to give 33.56 g (63.8%) of white crystalline product, m.p. 83—85°C.

Analysis:

Calculated for $C_{17}H_{15}NO_2S$: C, 68.66; H, 5.08; N, 4.71

Found: C, 68.51; H, 5.07; N, 4.73

Preparation 25

2-[3-(Phenylsulphonyl)propyl]-1H-isoindole-1,3-(2H)dione

To a solution of 30.21 g (0.102 mole) of 2-[3-(phenylthio)propyl]-1H-isoindole-1,3-(2H)dione (oil in Preparation 24) and 65.4 g of 80% (0.304 mole) metachloroperoxybenzoic acid in one litre of methylene chloride which had been stirred at room temperature for 5.5 hr was added a saturated aqueous solution of sodium carbonate. The mixture was stirred for 1/2 hr, the phases separated and the methylene chloride solution extracted with several portions of dilute sodium hydroxide solution. The methylene chloride layer was dried over magnesium sulphate and the solvent removed *in vacuo* to give an oil. Oil was added to a mixture of methylene chloride-hexane to give 27.56 g (82.1%) of white crystalline product, m.p. 126—127°C.

Analysis:

Calculated for $C_{17}H_{15}NO_4S$: C, 61.99; H, 4.59; N, 4.25

Found: C, 61.84; H, 4.61; N, 4.31

Preparation 26

N-(1-Methylethyl)-3-(phenylsulphonyl 1-propanamine hydrochloride

A solution of 66.55 g (0.3052 mole) of 3-chloropropylphenyl sulphone (prepared by reacting m-chloroperbenzoic acid and 3-chloropropyl phenylsulphide in methylene chloride) in 200 ml of isopropylamine was heated at 100°C overnight in a bomb. The isopropylamine was removed by rotary evaporation and the residue partitioned between chloroform and water. The chloroform layer was extracted with 1N sulphuric acid. The acidic layer was made alkaline and extracted with chloroform. The combined chloroform extract was evaporated to give an oil, the free base of the title compound. The oil was converted to the hydrochloride salt by reacting with ethereal hydrogen chloride. Recrystallization of the precipitated salt from methanol-diethyl ether gave 48.04 g (65.3%) of white crystalline powder, m.p. 185—186°C.

Analysis:

Calculated for $C_{12}H_{20}NO_2SCl$: C, 51.88; H, 7.26; N, 5.04

Found: C, 51.77; H, 7.27; N, 5.19

Preparation 27

N-[2-[(3,4-Dichlorophenyl)thio]ethyl]-N-(1-methylethyl)carbamic acid phenyl ester

To a solution of 29.9 g (0.1 mole) of N-[2-[3,4-dichlorophenyl)thio]ethyl]-2-propanamine and 10.1 g (0.1 mole) of triethylamine in 400 ml of benzene was added a benzene solution of phenyl chloroformate over a 1/2 hr period. The resulting solution was stirred overnight at room temperature. Chloroform was added and the solution extracted in sequence with water followed by 5% aqueous sodium hydroxide. The organic layer was extracted further with 1N sulphuric acid followed by dilute sodium hydroxide and then evaporated to give an oil. A portion of the oil was dried overnight *in vacuo* at 80°C.

Analysis:

Calculated for $C_{18}H_{19}NO_2SCl_2$: C, 56.25; H, 4.98; N, 3.64

Found: C, 55.89; H, 4.89; N, 3.58

Preparation 28

N-[2-[(3,4-Dichlorophenyl)sulphonyl]ethyl]-N-(1-methylethyl)carbamic acid phenyl ester

Following the general procedure of Preparation 32, N-[2-[(3,4-dichlorophenyl)thio]ethyl]-N-(1-methylethyl)carbamic acid phenyl ester was oxidized with metachloroperbenzoic acid to give the title compound.

Preparation 29

N-[2-[(3,4-Dichlorophenyl)sulphonyl]ethyl]-2-propanamine.

A solution of 44.27 g (0.107 mole) of N-[2-[(3,4-dichlorophenyl)sulphonyl]ethyl]-N-(1-methylethyl)carbamic acid phenyl ester in 300 ml of 48% HBr was heated at reflux for 12 hr. The reaction mixture was cooled to room temperature, made alkaline with 50% sodium hydroxide-ice and extracted with chloroform. The chloroform layer was extracted with 1N sulphuric acid. The sulphuric acid layer was extracted with chloroform and the chloroform layers combined and evaporated to an oil, the free base of the title compound. A portion of the oil was reacted with ethereal hydrogen chloride to give an overall yield of 32.4% of white crystalline product, m.p. 219—221°C.

Analysis:

| | | | | |
|---|---|---|---|---|
| Calculated for $C_{11}H_{15}NO_2SCl_3$ : | C, 39.84; | H, 4.56; | N, 4.22 |
| Found : | C, 39.62; | H, 4.90; | N, 4.19 |

Preparation 30

N-[2-[(4-Fluorophenyl)thio]ethyl]-2-propanamine hydrochloride.

A mixture of 70.0 g (0.546 mole) (p-fluorothiophenol, 44.0 g (0.546 mole) of 2-chloroethanol and 75.5 g (0.546 mole) of potassium carbonate in 800 ml of acetonitrile was heated overnight at reflux. The reaction mixture was filtered and stripped to dryness. The residue was then partitioned between aqueous sodium hydroxide and chloroform. Solvent was evaporated to give a light brown oil having NMR spectra corresponding to the desired 2-[(4-fluorophenyl)thio]ethanol. The oil was dissolved in 500 ml of benzene and to this solution was added 62.6 g (0.546 mole) of methane sulphonyl chloride over a 30 minute period with cooling using an ice bath. The resulting mixture was stirred overnight at room temperature, filtered and stripped to dryness. The residue was partitioned between 5% sodium hydroxide solution and chloroform. The chloroform layer was evaporated to dryness to give an oil comprising about 70% of the methane sulphonic acid ester of 2-[(4-fluorophenyl)thio]ethanol confirmed by NMR spectra. This crude mesylate (125 g) was stirred for five days at room temperature with 200 ml of isopropylamine. The reaction mixture was stripped to dryness and partitioned between chloroform and water. The chloroform layer was extracted with 1N sulphuric acid. The acidic layer was made alkaline and extracted with chloroform. The chloroform layers were combined and the solvent evaporated off to give a brown oil. The oil was reacted with ethereal hydrogen chloride to give the hydrochloride salt. Recrystallization from methanol-diethyl ether gave 41.5 g (30.5% based on p-fluorothiophenol) white crystalline product, m.p. 113.5—115°C.

Analysis:

| | | | |
|---|---|---|---|
| Calculated for $C_{11}H_{17}NSClF$ : | C, 52.90; | H, 6.86; | N, 5.61 |
| Found : | C, 52.92; | H, 6.88; | N, 5.70 |

Preparation 31

N-[2[(4-Fluorophenyl)thio]ethyl]-N-(1-methylethyl)carbamic acid phenyl ester.

To a solution of 38.5 g (0.15 mole) of N-[2-(4-fluorophenylthio]ethyl-1-methyl-ethanamine and 15.5 g (0.15 mole) of triethylamine in 300 ml of methylene chloride which was cooled in an ice bath was added dropwise with stirring a solution of 23.5 g (0.15 mole) of phenyl chloroformate in 100 ml of methylene chloride over a 15 minute period. The resulting solution was stirred overnight at room temperature and extracted with 5% aqueous sodium hydroxide. The chloroform layer was dried and filtered and evaporated to give an oil residue which crystallized to a white solid. A portion of the solid was triturated with isopropyl ether and the mixture cooled under refrigeration. The solid was collected by filtration and dried *in vacuo* overnight at 80°C. A white crystalline product, m.p. 53—58°C. was obtained.

Analysis:

| | | | |
|---|---|---|---|
| Calculated for $C_{28}H_{20}NO_2SF$ : | C, 64.84; | H, 6.05; | N, 4.20 |
| Found : | C, 64.97; | N, 6.06; | N, 4.13 |

Preparation 32

N-[2-[(4-Fluorophenyl)sulphonyl]ethyl]-N-(1-methylethyl)carbamic acid phenyl ester.

A mixture of 54.76 g (0.164 mole) of N-[2-[(4-fluorophenyl)thio]ethyl]-N-(1-methylethyl)carbamic acid phenyl ester and 179.64 g (0.832 mole) of metachloroperbenzoic acid was stirred overnight at room temperature. The resulting mixture was extracted with 5% sodium hydroxide followed by aqueous sodium sulphite. The methylene chloride contained a suspension of white solid which was dissolved by adding ethanol. The solution was dried and filtered and the solvent evaporated off to give an oil which crystallized

on standing. A portion of the solid was triturated with isopropyl ether and dried overnight *in vacuo* at 80°C to give a white crystalline solid, m.p. 94—95°C.

Analysis:

| | | | | | | | |
|---|---|---|---|---|---|---|---|
| Calculated for $C_{18}H_{20}NO_4SF$ | : | C, | 59.16; | H, | 5.52 | N, | 3.83 |
| Found | : | C, | 58.87; | N, | 5.39; | N, | 3.57 |

Preparation 33

N-[2-[(4-Fluorophenyl)sulphonyl]ethyl]-2-propanamine hydrochloride.

A solution of 60.25 g (0.165 mole) of N-[2-[(4-fluorophenyl)sulphonyl]ethyl]-N-(1-methylethyl)carbamic acid phenyl ester in 400 ml of 48% HBr was heated at reflux for 8 hr. The reaction mixture was cooled with ice and made alkaline with 50% sodium hydroxide. The aqueous phase was extracted with chloroform. The chloroform layer was extracted with sodium hydroxide. Evaporation of the chloroform layer gave an oil residue. The oil was dissolved in methylene chloride and the solution extracted with 1N sulphuric acid. The acidic layer was made alkaline with a mixture of ice and 50% sodium hydroxide solution and extracted with chloroform. Removal of chloroform gave an oil, the free base of the title compound, which was dissolved in methanol and reacted with ethereal hydrogen chloride to give the hydrochloride salt. On recrystallization from methanol-diethyl ether a white crystalline product; m.p. 168—169°C 31.1% yield was obtained.

Analysis:

| | | | | | | | |
|---|---|---|---|---|---|---|---|
| Calculated for $C_{11}H_{17}NO_2SF$ | : | C, | 46.89; | H, | 6.08; | N, | 4.97 |
| Found | : | C, | 46.71; | H, | 6.09; | N, | 4.98 |

Preparation 34

N-[2-[(4-Methoxyphenyl)sulphonyl]ethyl]-2-propanamine hydrochloride.

A solution of 16.46 g (0.055 mole) of N-[2-[(4-chlorophenyl)sulphonyl]ethyl-1-methyl ethanamine hydrochloride and 16.2 g (0.3 mole) of sodium methylate in 500 ml of dimethylsulphoxide was heated at 95°C for 2 hr with stirring. The solvent was removed on a rotary evaporator at reduced pressure and the residue was partitioned between water and chloroform. The aqueous phase was made strongly alkaline with 50% sodium hydroxide and extracted with chloroform. The chloroform layers were combined and evaporated to leave an oil. The oil was reacted with ethereal hydrogen chloride. Recrystallization of the salt from methanol-diethyl ether gave 9.11 g (56.4%) of a white crystalline product, m.p. 142—145°C.

Analysis:

| | | | | | | | |
|---|---|---|---|---|---|---|---|
| Calculated for $C_{12}H_{20}NO_3SCl$ | : | C, | 49.06; | H, | 6.86; | N, | 4.77 |
| Found | : | C, | 48.98; | H, | 6.91; | N, | 4.80 |

Preparation 35

2-[2-(Phenylsulphonyl)ethyl]-1H-isoindole-1,3-(2H)-dione.

A solution of 30.7 g (0.15 mole) of 2-chloroethylphenylsulphone and 69.5 g (0.375 mole) of potassium phthalimide in 600 ml of dimethylformamide was heated overnight at 85°C. The reaction mixture was stripped to dryness and the residue was partitioned between water and chloroform. The chloroform layer was dried and filtered and the solvent was evaporated off from the filtrate to give an oil which crystallized on standing. The solid was triturated with isopropyl ether and the mixture cooled under refrigeration. The solid was collected by filtration and dried and recrystallized from methanol-isopropyl ether to give white crystalline product, m.p. 186—188°C., in 35.7% yield.

Analysis:

| | | | | | | | |
|---|---|---|---|---|---|---|---|
| Calculated for $C_{16}H_{13}NO_4S$ | : | C, | 60.94;I | H, | 4.16; | N, | 4.44 |
| Found | : | C, | 60.70; | H, | 4.13; | N, | 4.42 |

Preparation 36

2-(Phenylsulphonyl)ethanamine hydrochloride.

A solution of 32.3 g (0.102 mole) of 2-[2-(phenylsulphonyl)ethyl]-1H-isoindole-1,3-(2H)-dione and 11.8 g (0.2 mole) of 85% hydrazine hydrate in 500 ml of absolute ethanol was refluxed for 6 hr. The reaction mixture was cooled to room temperature, filtered and concentrated. The concentrate was dissolved in chloroform and extracted with 5% sodium hydroxide. The chloroform layer was dried and evaporated to dryness to give a clear oil. The oil, the free base of the title compound, was reacted with ethereal hydrogen chloride. Recrystallization of the precipitated salt from methanol-diethyl ether gave a white crystalline product, m.p. 151—154°C in 40.5% yield.

Analysis:

| | | | | | | | |
|---|---|---|---|---|---|---|---|
| Calculated for $C_8H_{12}NO_2SCl$ | : | C, | 43.34; | N, | 5.46; | N, | 6.32 |
| Found | : | C, | 43.09; | H, | 5.44; | N, | 6.42 |

Preparation 37

N-[2-[(4-Chlorophenyl)thio]ethyl-N-(1-methylethyl)carbamic acid phenyl ester.

To a solution of 22.8 g (0.1 mole) of N-[2-[(4-chlorophenyl)thio]ethyl]-1-methylethanamine (oil in Preparation 1) and 10.1 g (0.1 mole) of triethylamine in 300 ml of methylene chloride was added dropwise with stirring a solution of 15.7 g (0.1 mole) phenyl chloroformate in 100 ml of methylene chloride. The

resulting solution was stirred overnight at room temperature. The methylene chloride layer was extracted with 5% sodium hydroxide solution followed by 1N sulphuric acid. The methylene chloride layer was concentrated to give an oil which crystallized to a white solid. Recrystallization from isopropyl ether gave white crystalline product, m.p. 54—56°C in 79.6% yield.

Analysis:

| | | | | |
|---|---|---|---|---|
| Calculated for $C_{18}H_{20}NO_2SCl$ : | C, 61.79; | H, 5.76; | N, 4.00 |
| Found : | C, 61.77; | H, 5.77; | N, 3.98 |

Preparation 38

N-[2-[(4-Chlorophenyl)sulphonyl]ethyl]-N-(1-methylethyl)carbamic acid phenyl ester.

To a cold solution of 33.88 g (0.089 mole) of N-[2-[(4-chlorophenyl)thio]ethyl]-N-(1-methylethyl)carbamic acid phenyl ester in one litre of methylene chloride was added 120.8 g (0.7 mole) of solid m-chloroperoxybenzoic acid in portions over a 20 minute period. The mixture was stirred overnight at room temperature. The methylene chloride layer was extracted with 5% sodium hydroxide and sodium bisulphite. The methylene chloride layer was concentrated to an oil which crystallized on standing. Recrystallization from isopropanol ether gave white crystalline product, m.p. 77.5—79.0°C in 85.3% yield.

Preparation 39

N-[2-[(4-Chlorophenyl)sulphonyl]ethyl]-1-methylethanamine hydrochloride.

When in the procedure of Preparation 29, N-[2-[(4-chlorophenyl]sulphonyl]ethyl]-N-(1-methylethyl)carbamic acid phenyl ester was substituted for N-[2-[(3,4-dichlorophenyl)sulphonyl]ethyl]-N-(1-methylethyl)carbamic acid phenyl ester, the title compound was obtained.

Preparation 40

2-[Methyl-(2-phenylethyl)amino]acetonitrile.

A solution of N-methylphenethylamine, chloroacetonitrile and excess triethylamine in absolute ethanol was refluxed for 16 hr. The solvent was removed *in vacuo* and the residue was partitioned between methylene chloride and water. The methylene chloride solution was extracted with several portions of dilute sulphuric acid. The acidic extract is made basic with 50% sodium hydroxide and the basic mixture was extracted with methylene chloride. The methylene chloride solution was dried over magnesium sulphate and the solvent was removed *in vacuo* to give the title compound.

Preparation 41

N-Methyl-N-(2-phenylethyl)-1,2-ethanediamine, maleate.

A mixture of 2-[methyl(phenylethyl)amino]acetonitrile and excess lithium aluminium hydride in tetrahydrofuran was stirred at room temperature for 4 hrs. Dilute aqueous sodium hydroxide was added slowly to the reaction mixture until evolution of hydrogen gas had ceased. The resulting mixture was filtered and the solvent was removed from the filtrate *in vacuo.* The residue was partitioned between methylene chloride and water. The methylene chloride solution was dried over magnesium sulphate and the solvent was removed *in vacuo* to give the free base of the title compound. The free base was reacted with maleic acid to give the product.

Preparation 42

2-[Methyl(phenylmethyl)amino]acetonitrile.

When in the procedure of Preparation 40, benzylmethylamine was substituted for N-methylphenethylamine, the title compound was obtained.

Preparation 43

N-Methyl-N-(phenylmethyl)-1,2-ethanediamine maleate.

When in the procedure of Preparation 41, 2-[methyl(phenylmethyl)amino]acetonitrile was substituted for 2-[methyl-(2-phenylethylamino]acetonitrile, the title compound was obtained.

Preparation 44

N-Methyl-2-(phenylsulphonyl)ethanamine.

A solution of 2-chloroethyl phenyl sulphone and a large excess of methylamine (40% solution in water) in acetonitrile was stirred at room temperature overnight. The solvent is removed *in vacuo* and the residue was partitioned between methylene chloride and dilute sodium hydroxide. The methylene chloride solution was extracted with dilute sulphuric acid. The acidic extract was made basic with 50% sodium hydroxide and the basic solution was extracted with methylene chloride. The methylene chloride solution was dried over magnesium sulphate and the solvent was removed *in vacuo* to give the title compound.

Preparation 45

N-[2-[(2-Furanylmethyl)thio]ethyl]-2-propanamine hydrochloride.

When in the procedure of Preparation 1, 2-chloroethyl furanylmethyl sulphide was substituted for 2-chloroethyl-p-chlorophenyl sulphide, the title compound was obtained.

Preparation 46

N-[2-[(2-Furanylmethyl)sulphinyl]ethyl]-2-propanamine.

When in the procedure of Preparation 3, N-[2-[furanylmethyl)thio]ethyl]-2-propanamine was substituted for N-[2-(4-chlorophenyl)thio]ethyl]-1-methylethylanamine, the title compound was obtained.

Preparation 47

N-[2-[(Phenylmethyl)thio]ethyl]-2-propanamine hydrochloride.

When in the procedure of Preparation 1, benzyl 2-chloroethyl sulphide was substituted for 2-chloroethyl-p-chlorophenyl sulphide, the title compound was obtained.

Preparation 48

N-[2-[(Phenylmethyl)sulphinyl]ethyl]-2-propanamine.

When in the procedure of Preparation 3, N-[2-[(phenylmethyl)thio]ethyl]-2-propanamine was substituted for N-[2-[(4-chlorophenyl)thio]ethyl]-1-methylethanamine, the title compound was obtained.

Preparations 49a to e

Following the procedure of Preparation 5, substituting the following for 2-chloroethyl-p-chlorophenyl sulphone:

2-chloroethyl p-bromophenyl sulphone,
2-chloroethyl 4-*t*-butylphenyl sulphone,
2-chloroethyl 2-furanylmethyl sulphone,
2-chloroethyl benzyl sulphone, and
2-chloroethyl 3-(trifluoromethyl)phenyl sulphone, there were obtained:
a) N-[2-[(4-bromophenyl)sulphonyl]ethyl]-2-propanamine,
b) N-[2-[(4-*t*-butylphenyl)sulphonyl]ethyl]-2-propanamine,
c) N-[2-[(2-furanylmethyl)sulphonyl]ethyl]-2-propanamine,
d) N-[2-[(phenylmethyl)sulphonyl]ethyl]-2-propanamine, and
e) N-[2-[[3-(trifluoromethyl)phenyl]sulphonyl]ethyl]-2-propanamine.

The following examples 1 to 60 serve to illustrate the preparation of compounds useful in treating arrhythmias in the method of the present invention. The scope of the invention is, however, not limited thereto. Structures are illustrated in Table 1.

Example 1

N-[2-[(4-Chlorophenyl)thio]ethyl]-N'-[2-(dimethylamino)ethyl]-N-(1-methylethyl)urea, fumarate [1:1.5].

To a solution of 25 ml of 12.5% phosgene in benzene (0.0475 mole) and 6.42 g Proton Sponge (Registered Trade Mark) [which is 1,8-bis-(dimethylamino)naphthalene) (0.03 mole) in 300 ml of methylene chloride was added a methylene chloride solution containing 6.87 g (0.03 mole) of N-[2-[(4-chlorophenyl)thio]ethyl]-1-methylethanamine (oil in Preparation) over a 45 min period. The solution was stirred for 2.5 hr at room temperature and extracted with 1N aqueous sulphuric acid solution. The methylene chloride layer was dried over anhydrous potassium carbonate. The organic solvent was removed in a rotary evaporator to give an oil residue which was then dissolved in tetrahydrofuran. To this solution was added 5.28 g (0.06 mole) of N,N-dimethylethylenediamine (unsym-dimethyl ethylenediamine). The reaction mixture was stripped to dryness and the residue partitioned between water and chloroform followed by washing of the chloroform layer several times with water. The chloroform layer was evaporated to give a yellow oil, the free base of the title compound, which was reacted with fumaric acid to give crystalline solid. Recrystallization from methanol-diethyl ether gave 6.86 g (44.1%) of white crystalline product, m.p. 101—103.5°C.

Analysis:

| | | | | | | | |
|---|---|---|---|---|---|---|---|
| Calculated for $C_{22}H_{32}N_3O_7SCl$ : | C, | 51.00; | H, | 6.23; | N, | 8.11 |
| Found : | C, | 50.65; | H, | 6.20; | N, | 8.17 |

Example 2

N'-[2-(Dimethylamino)ethyl]-N-(1-methylethyl)-N-[2-[(naphthylene)thio]ethyl]urea succinate [1:1.5].

The title compound was prepared by Method A and the procedure of Example 1, reacting in sequence:

0.0475 mole phosgene; Proton Sponge

5.81 g (0.0237 mole) of 1-methyl-N-[2-(2-naphthylenylthio)ethyl]ethanamine (from neutralizing the hydrochloride obtained in Preparation 2),

4.40 g (0.05 mole) unsym-N,N-dimethylethylenediamine to give an oil, the free base of the title compound, which was then reacted with succinic acid, (45.3%), m.p. 96—98°C.

Analysis:

| | | | | | | |
|---|---|---|---|---|---|---|
| Calculated for $C_{26}H_{38}N_3O_7S$ : | C, | 58.19; | H, | 7.14; | N, | 7.83 |
| Found : | C, | 58.06; | H, | 7.16; | N, | 7.75 |

Example 3

N-[2-[(4-Chlorophenyl)sulphonyl]ethyl]-N′-[2-(dimethylamino)ethyl]-N-(1-methylethyl)urea maleate [1:1].

The title compound was prepared by Method A and the procedure of Example 1, reacting in sequence:

0.0475 mole phosgene, Proton Sponge,

6.53 g (0.025 mole) of N-[2-[(4-chlorophenyl)sulphonyl]ethyl]-1-methylethanamine (oil in Preparation 5),

4.20 g (0.05 mole) unsym-dimethylethylenediamine to give an oil, the free base of the title compound which was then reacted with maleic acid (73.5%), m.p. 134—135°C.

Analysis:

| | | | | |
|---|---|---|---|---|
| Calculated for $C_{20}H_{30}N_3O_7ClS$ | : | C, 48.83; | H, 6.15; | N, 8.54 |
| Found | : | C, 48.76; | H, 6.15; | N, 8.57 |

Example 4

N-[2-[(4-Chlorophenyl)sulphonyl]ethyl]-N′-[2-(diethylamino)ethyl]-N-(1-methylethyl)urea tartrate [1:1].

The title compound was prepared by Method A and the procedure of Example 1, reacting in sequence:

0.060 mole phosgene, Proton Sponge,

10.44 g (0.04 mole) of N-[2-[(4-chlorophenyl)sulphonyl]ethyl]-1-methylethanamine (oil in Preparation 5),

9.28 g (0.08 mole) unsym-diethylethylenediamine to give an oil, the free base of the title compound which was then reacted with tartaric acid (55.8%), m.p. 135—137°C.

Analysis:

| | | | | |
|---|---|---|---|---|
| Calculated for $C_{22}H_{36}N_3O_9SCl$ | : | C, 47.69; | H, 6.65; | N, 7.58 |
| Found | : | C, 47.75; | H, 6.61; | N, 7.58 |

Example 5

N-[2-[(4-Chlorophenyl)sulphonyl]ethyl]-N′-[2-(dimethylamino)ethyl]-N′-(1-methylethyl)urea maleate [1:1].

The title compound was prepared by Method A and the procedure of Example 1, reacting in sequence:

0.1014 mole phosgene, Proton Sponge,

9.97 g (0.0455 mole) of 2-[(4-chlorophenyl)sulphonyl]ethanamine (oil in Preparation 8),

5.05 g (0.05 mole) triethylamine,

5.92 g (0.046 mole) of N-isopropyl-N′, N′-dimethylenediamine to give an oil which crystallized to a brown solid, the free base of the title compound which was then reacted with maleic acid to give the title compound (41.2%), m.p. 125—126.5°C.

Analysis:

| | | | | |
|---|---|---|---|---|
| Calculated for $C_{20}H_{30}N_3O_7SCl$ | : | C, 48.83; | H, 6.15; | N, 8.54 |
| Found | : | C, 48.64; | H 6.16; | N, 8.58 |

Example 6

N-[2-[(4-Chlorophenyl)sulphonyl]ethyl]-N′-[2-(dimethylamino)ethyl]-N-(1-methylethyl)thiourea, hemifumarate.

The title compound was prepared by Method A and the procedure of Example 1 with modification as indicated, reacting in sequence:

4.90 g (0.043 mole) thiophosgene, Proton Sponge,

7.83 g (0.03 mole) of N-[2-[(4-chlorophenyl)sulphonyl]ethyl]-1-methylethanamine (oil in Preparation 5),

5.28 g (0.06 mole) unsym-N,N-dimethylethylenediamine, to give an oil which was subjected to column chromatography on a silica-gel column, eluting with 5—95 (methanol-chloroform). The pure fractions were combined, solvent removed and an oil, the free base of the title compound, obtained which was reacted with fumaric acid to give the title compound (16.6%), m.p. 141—142°C.

Analysis:

| | | | | |
|---|---|---|---|---|
| Calculated for $C_{18}H_{28}N_2O_4S_2Cl$ | : | C, 48.04; | H, 6.27; | N, 9.34 |
| Found | : | C, 47.74; | H, 6.21; | N, 9.35 |

Example 7

N-[2-[(4-Chlorophenyl)sulphinyl]ethyl]-N′-[2-(dimethylamino)ethyl]-N-(1-methylethyl)urea, fumarate [1:1].

(Demonstration of Method B)

To a solution of 4.70 g (0.029 mole) of 1,1′-carbonyldiimidazole 2.38 g (0.027 mole) of N,N-dimethylaminoethylamine in tetrahydrofuran which had been stirred at room temperature for 50 min was added a solution of 6.14 g (0.0251 mole) of N-[2-[(4-chlorophenyl)sulphinyl]ethyl]-1-methylethanamine in tetrahydrofuran. The solution was refluxed for about 10 hr and the solvent removed *in vacuo* to give an oil residue. The oil was dissolved in methylene chloride and washed by extraction several times with water. The methylene chloride solution was dried over magnesium sulphate and evaporated *in vacuo* to give an oil, the free base of the title compound. A solution of the oil in methanol was reacted with fumaric acid and the salt precipitated by addition of diethyl ether as white crystalline solid (58.6%), m.p. 112.5—114.5°C.

Analysis:

| | | | | |
|---|---|---|---|---|
| Calculated for $C_{20}H_{30}N_3O_6SCl$ | : | C, 50.47; | H, 6.35; | N, 8.83 |
| Found | : | C, 50.45; | H, 6.38; | N, 8.86 |

## Example 8

N'-[2-(Dimethylamino)ethyl]-N-[1-methylethyl)-N-[2-(phenylsulphonyl)ethyl]urea, maleate [1:1].

The title compound was prepared by Method B and the procedure of Example 7, reacting in sequence:

5.67 g (0.035 mole) of 1,1-carbonyldiimidazole,

2.91 g (0.033 mole) of N,N-dimethylaminoethylamine,

6.00 g (0.026 mole) of 1-methyl-N-[2-(phenylsulphonyl)ethyl]ethanamine (oil in Preparation 6) to give an oil, the free base of the title compound which was then reacted with maleic acid (39.2%), m.p. 103—105°C.

Analysis:

| | | | | |
|---|---|---|---|---|
| Calculated for $C_{20}H_{31}N_3O_7S$ | : | C, 52.50; | H, 6.83; | N, 9.18 |
| Found | : | C, 52.48; | H, 6.90; | N, 9.18 |

## Example 9

N'-[2-(Diethylamino)ethyl]-N-(1-methylethyl)-N-[2-(phenylsulphonyl)ethyl]urea maleate [1:1].

The title compound was prepared by Method B and the procedure of Example 7, reacting in sequence:

9.72 g (0.060 mole) of 1,1'-carbonyldiimidazole,

5.19 g (0.045 mole) of N,N-diethylaminoethylamine, and

10.33 g (0.046 mole) of 1-methyl-N-[2-(phenylsulphonyl)ethyl]ethanamine (oil of Preparation 6) to give an oil, the free base of the title compound which was then reacted with maleic acid, m.p. 88—90°C.

Analysis:

| | | | | |
|---|---|---|---|---|
| Calculated for $C_{22}H_{35}N_3O_7S$ | : | C, 54.42; | H, 7.27; | N, 8.65 |
| Found | : | C, 54.33; | H, 7.30; | N, 8.63 |

## Example 10

N'-[2-(Dimethylamino)ethyl]-N-(1-methylethyl)-N-[2-[(1-naphthalenesulphonyl)ethyl]urea, maleate.

To a solution of 35 ml of 12.5% phosgene in benzene (0.0665 mole) and 9.0 g Proton Sponge [1,8-bis-(dimethylamino)naphthalene] (0.02 mole) in 200 ml of methylene chloride was added a solution of 4.60 g (0.0166 mole) of 1-methyl-N-[2-[(1-naphthalenyl)sulphonyl]ethyl]ethanamine obtained as a brown oil in Preparation 12) in 100 ml methylene chloride over a 20 minute period. The reaction mixture was stirred for one hr at room temperature and then extracted with 1N sulphuric acid. The methylene chloride layer was separated, dried over potassium carbonate, filtered and evaporated. The yellow crystalline solid obtained was dissolved in 350 ml of tetrahydrofuran. To this solution was added 3.52 g (0.04 mole) of unsym-dimethylethylenediamine and the mixture was stirred overnight at room temperature. The reaction mixture was stripped to dryness and the residue partitioned between chloroform and water. Evaporation of the chloroform layer gave a dark brown oil, the free base of the title compound which was reacted with maleic acid. The maleate salt was recrystallized from methanol-diethyl ether to give 5.51 g (65.4%) of yellow solid, m.p. 126—128°C.

Analysis:

| | | | | |
|---|---|---|---|---|
| Calculated for $C_{24}H_{33}N_3O_7S$ | : | C, 56.79; | H, 6.55; | N, 8.28 |
| Found | : | C, 56.60; | H, 6.61; | N, 8.26 |

## Example 11

N'-[2-(Diethylamino)ethyl]-N-(1-methylethyl)-N-[2-(2,3-dihydro-1H-inden-4-yl-sulphonyl)ethyl]urea, maleate.

The title compound was prepared by Method B, reacting in sequence:

1,1'-carbonyldiimidazole,

unsym-N,N-diethylethylendiamine, and

1-methyl-N-[2-(2,3-dihydro-1H-inden-4-yl-sulphonyl)]ethanamine, to give the free base of the title compound which was then reacted with maleic acid to give the title compound.

## Example 12

N'-[2-(Diethylamino)ethyl]-N-(1-methylethyl)-N-[2-(2,3-dihydro-1H-inden-5-yl-sulphonyl)ethyl]urea, maleate.

The title compound was prepared by Method B, reacting in sequence:

1,1'-carbonyldiimidazole,

unsym-N,N-diethylethylenediamine and

1-methyl-N-[2-(2,3-dihydro-1H-inden-5-yl-sulphonyl)]ethanamine, to give the free base of the title compound which was then reacted with maleic acid to give the title compound.

## Example 13

N'-[2-(Diethylamino)ethyl]-N-(1-methylethyl)-N-[2-[(4-methylphenyl)sulphonyl]ethyl]urea maleate.

The title compound was prepared by Method B, reacting in sequence:

1,1'-carbonyldiimidazole,

unsym-N,N-diethylethylenediamine, and

N-[2-[(4-methylphenyl)sulphonyl]ethyl]-1-methylethanamine, to give the free base of the title compound which was then reacted with maleic acid to give the title compound.

Example 14

N'-[2-(Diethylamino)ethyl]-N-(1-methylethyl)-N-[2-[(methoxyphenyl)sulphonyl]ethyl]urea, maleate.

The title compound was prepared by Method B, reacting in sequence:
1,1'-carbonyldiimidazole,
unsym-N,N-diethylethylenediamine, and
N-[2-[(4-methoxyphenyl)sulphonyl]ethyl]-1-methylethanamine, to give the free base of the title compound which was then reacted with maleic acid to give the title compound.

Example 15

N-[2-[(3,5-Dichlorophenyl)sulphonyl]ethyl]-N'-[2-(diethylamino)ethyl]-N-(1-methylethyl)urea, maleate.

The title compound was prepared by Method B, reacting in sequence:
1,1'-carbonyldiimidazole,
unsym-N,N-diethylenediamine, and
N-[2-[(3,5-dichlorophenyl)sulphonyl]ethyl]-1-methylethanamine, to give the free base of the title compound which was then reacted with maleic acid to give the title compound.

Example 16

N'-[2-(Diethylamino)ethyl]-N-[2-[3,4,5-trimethoxyphenyl)sulphonyl]ethyl]-N-(1-methylethyl)urea, maleate.

The title compound was prepared by Method B, reacting in sequence:
1,1'-carbonyldiimidazole,
unsym-N,N-diethylethylenediamine, and
N-[2[(3,4,5-trimethoxyphenyl)sulphonyl]ethyl]-1-methylethanamine, to give the title compound which then reacted with maleic acid to give the title compound.

Example 17

N'-[2-(Dimethylamino)ethyl]-N-[2-[(4-fluorophenyl)sulphonyl]ethyl]-N-(1-methylethyl)urea, maleate.

The title compound was prepared by Method B, reacting in sequence:
1,1'-carbonyldiimidazole,
unsym-N,N-dimethylethylenediamine, and
N-[2-[(4-fluorophenyl)sulphonyl]ethyl]-1-methylethanamine, to give the free base of the title compound which was then reacted with maleic acid to give the title compound.

Example 18

N'-[2-(Diethylamino)ethyl]-N-(1-methylethyl)-N-[2-[(4-trifluoromethylphenyl)sulphonyl]ethyl]urea, maleate.

The title compound was prepared by Method B, reacting in sequence:
1,1'-carbonyldiimidazole,
unsym-N,N-diethylethylenediamine, and
1-methyl-N-[2-[(4-trifluoromethylphenyl)sulphonyl]ethyl]ethanamine, to give the free base of the title compound which was then reacted with maleic acid to give the title compound.

Example 19

N-[2-[(4-Cyanophenyl)sulphonyl]ethyl]-N'-[2-(diethylamino)ethyl]-N-(1-methylethyl)urea, maleate.

The title compound was prepared by Method B, reacting in sequence:
1,1'-carbonyldiimidazole,
unsym-N,N-diethylethylenediamine, and
N-[2-[(cyanophenyl)sulphonyl]ethyl]ethanamine, to give the free base of the title compound which was then reacted with maleic acid to give the title compound.

Example 20

N-[3-[(4-Chlorophenyl)sulphonyl]propyl]-N'-[2-(diethylamino)ethyl]-N'-(1-methylethyl)urea, maleate.

The title compound was prepared by Method C, reacting in sequence:
1,1'-carbonyldiimidazole,
3-[(4-chlorophenyl)sulphonyl]propanamine (free base in Preparation 10 prepared by neutralization), and
N-isopropyl-N',N'-diethylethylenediamine, to give the free base of the title compound which was then reacted with maleic acid to give the title compound.

Example 21

N'-[2-(Diethylamino)ethyl]-N'-(1-methylethyl)-N-[3-(phenyl sulphonyl)propyl]urea, maleate.

The title compound was prepared by Method C, reacting in sequence:

1,1'-carbonyldiimidazole,

3-(phenylsulphonyl)propanamine, and

N-isopropyl-N',N'-diethylethylenediamine, to give the free base of the title compound which was then reacted with maleic acid to give the title compound.

Example 22

N'-[3-(Dimethylamino)propyl]-N-(1-methylethyl)-N-[2-(phenylsulphonyl)ethyl]urea, maleate.

The title compound was prepared by Method B, reacting in sequence:

1,1'-carbonyldiimidazole,

unsym-N,N-dimethylpropylenediamine, and

1-methyl-N-[2-(phenylsulphonyl)ethyl]ethanamine (oil in preparation 6), to give the free base of the title compound which was then reacted with maleic acid to give the title compound.

Example 23

N-(1-Methylethyl)-N-[2(phenylsulphonyl)ethyl]-N'-[2-(1-pyrrolidinyl)ethyl]urea oxalate.

The title compound was prepared by Method B, reacting in sequence:

N-(2-aminoethyl)pyrrolidine,

1,1'-carbonyldiimidazole, and

1-methyl-N-[2-(phenyl sulphonyl)ethyl]ethanamine (oil in preparation 6), to give the free base of the title compound which was then reacted with oxalic acid.

Examples 24A to 24C

Following the procedure of Example 23 and substituting the following for N-(2-aminoethyl)pyrrolidine:

N-(2-aminoethyl)piperidine,

N-(2-aminoethyl)morpholine, and

N-(2-aminoethyl)-4-methylpiperazin-1-yl, there were obtained:

N-(1-methylethyl)-N-[2-(phenylsulphonyl)ethyl]-N'-[2-(1-piperidinyl)ethyl urea, oxalate, (Example 24A)

N-(1-methylethyl)-N-[2-(phenylsulphonyl)ethyl]-N'-[2-(4-morpholino)ethyl urea, oxalate, (Example 24B), and

N-(1-methylethyl)-N-[2-(phenylsulphonyl)ethyl]-N'-[2-(4-methylpiperazin-1-yl)ethyl urea, oxalate (Example 24C).

Example 25

N-Cyclohexyl-N'-[2-(diethylamino)ethyl]-N-[2-phenylsulphonyl)ethyl]urea, maleate.

The title compound was prepared by Method B and the procedure of Example 7 by reacting in sequence:

1,1'-carbonyldiimidazole,

N,N-diethylaminoethylamine, and

N-cyclohexyl-N-[2-(phenylsulphonyl)ethyl]amine, to give the free base of the title compound which was then reacted with maleic acid to give the title compound.

Example 26

N'-[2-(Diethylamino)ethyl]-N-phenyl-N-[2-(phenylsulphonyl)ethyl]urea, maleate.

The title compound was prepared by Method B and the procedure of Example 7 by reacting in sequence:

1,1'-carbonyldiimidazole,

N,N-diethylaminoethylamine, and

N-phenyl-N-[2-(phenylsulphonyl)ethyl]amine, to give the free base of the title compound which was then reacted with maleic acid to give the title compound.

Example 27

N-Benzyl-N'-[2-(diethylamino)ethyl]-N-[2-(phenylsulphonyl)ethyl]urea, maleate.

The title compound was prepared by Method B and the procedure of Example 8 by reacting in sequence:

1,1'-carbonyldiimidazole,

N,N-diethylaminoethylamine, and

N-benzyl-N-[2-(phenyl sulphonyl)ethyl]amine, to give the free base of the title compound which was then reacted with maleic acid to give the title compound.

Example 28

N-(1-Methylethyl)-N-[2-(phenylsulphonyl)ethyl]-N'-(2-aminoethyl)urea.

The title compound was prepared by Method A, reacting in sequence:
phosgene,
Proton Sponge,
1-methyl-N-[2-(phenyl sulphonyl)ethyl]ethanamine (oil in preparation 6), and ethylenediamine, to give the free base of the title compound.

Example 29

N'-[2-(Methylamino)ethyl]-N'-methyl-N-(1-methylethyl)-N-[2-phenyl sulphonyl)ethyl]urea, maleate.

The title compound was prepared by Method A by reacting in sequence:
phosgene,
Proton Sponge,
1-methyl-N-[2-phenylsulphonyl)ethyl]ethanamine (oil in preparation 6), and
sym-N,N-dimethylethylenediamine, to give the free base of the title compound which was then reacted with maleic acid to give the title compound.

Example 30

N'-[2-(Diethylamino)ethyl]-N-(1-methylethyl)-N-[2-phenylsulphonyl)ethyl]thiourea, fumarate.

The title compound was prepared following the procedure of Example 6, reacting in sequence:
thiophosgene, Proton Sponge,
1-methyl-N-[2-(phenyl sulphonyl)ethyl]ethanamine (oil in preparation 6), and
unsym.-N,N-diethylethylenediamine, to give the free base of the title compound which was reacted with fumaric acid to give the title compound.

Example 31

N'-[2-(Dimethylamino)ethyl]-N-(1-methylethyl)-N-[2-[(4-methylphenyl)sulphonyl]ethyl]urea, maleate.

The title compound was prepared by Method A and the procedure of Example 1 by reacting in sequence:
phosgene, Proton Sponge,
1-methyl-N-[2-[(4-methylphenyl)sulphonyl]ethyl]ethanamine (oil obtained in preparation 14),
unsym-N,N-dimethylethylenediimine, to give an oil, the free base of the title compound which was then reacted with maleic acid to give the title compound.

Example 32
(Method C)

N-[2-[(4-Chlorophenyl)sulphonyl]ethyl]-N'-[2-(dimethylamino)ethyl]-N'-(1-methylethyl)urea maleate.

A tetrahydrofuran solution of equal molar amounts of 1,1'-carbonyldiimidazole and 3-[(4-chlorophenyl)sulphonyl]ethanamine (free base in preparation 10) was stirred at room temperature for several hours. N-Isopropyl-N',N'-dimethylenediamine in 50% molar excess was added and the mixture was heated with stirring under reflux for several hours. The tetrahydrofuran was removed and the resultant oil partitioned between chloroform and water. The free base was isolated by evaporation of the chloroform layer and thereafter reacted with maleic acid to give the title compound.

Example 33
(Method D)

N-[3-[(4-Chlorophenyl)sulphonyl]propyl]-N'-(1-methylethyl)-N'-[2-(dimethylamino)ethyl]urea, maleate.

To a solution of phosgene in methylene chloride was added an equimolar amount of N'-isopropyl-N,N-dimethylethylenediamine also in methylene chloride over a 30 minute period. The solution was stirred for one hour at room temperature. To the reaction mixture was added dropwise with stirring a molar equivalent amount of 3-[(4-chlorophenyl)sulphonyl]propanamine (free base in preparation 10) and a double molar portion of triethylamine over a 30 min. period. The reaction mixture was stirred overnight at room temperature and thereafter extracted with aqueous 10% sodium hydroxide solution. The methylene chloride layer was extracted with 1N sulphuric acid. The acid layer was made alkaline and extracted with chloroform. The chloroform layer was evaporated to give the free base of the title compound. The free base was converted to the maleate salt by reaction with maleic acid and recrystallized by use of conventional solvents.

Example 34

N'-[2-(Dimethylamino)ethyl]-N-(1-methylethyl)-N-[2-[(1-naphthaleneylthio)]ethyl]urea, maleate.

The title compound was prepared by Method A and the procedure of Example 10, reacting in sequence:
phosgene,
Proton Sponge,
1-methyl-N-[2-(1-naphthalenylthio)ethyl]ethanamine (free base in preparation 11), and
unsym-dimethylethylenediamine, to give the free base of the title compound which was then reacted with maleic acid to give the title compound.

Example 35

N'-[2-(Dimethylamino)ethyl]-N-(1-methylethyl)-N-[2-[(1-naphthalenesulphinyl)]ethyl]urea maleate.

The title compound was prepared by Method B and the procedure of Example 7, reacting in sequence:
1,1'-carbonyldiimidazole,
unsym-dimethylethylenediamine, and
1-methyl-N-[2-(1-naphthalenesulphinyl)ethyl]ethanamine (free base in Preparation 18) to give the free base of the title compound which was then reacted with maleic acid to give the title compound.

Example 36

N'-[2-(Dimethylamino)ethyl]-N-(1-methylethyl)-N-[2-[(4-nitrophenylsulphonyl)ethyl]urea, maleate.

The title compound was prepared by Method B, reacting in sequence:
1,1'-carbonyldiimidazole,
unsym-N,N-dimethylethylenediamine, and
N-[2-[(4-nitrophenyl)sulphonyl]ethyl]-1-methylethanamine from Preparation 16, to give the free base of the title compound which was then reacted with maleic acid to give the title compound.

Example 37

N'-[2-(Dimethylamino)ethyl]-N-(1-methylethyl)-N-[2-(1-naphthalenylthio)ethyl]urea, oxalate.

To a solution of 7.0 g (0.0327 mole) of Proton Sponge and phosgene (80 ml benzene solution of 12.5% phosgene) in 400 ml of methylene chloride was added a methylene chloride solution of 8.03 g (0.0327 mole) of 1-methyl-N-[2-(1-naphthalenylthio)ethyl]ethanamine (oil in Preparation 11). The resulting solution was stirred for 2.5 hr at room temperature, then extracted with 1N sulphuric acid solution. The methylene chloride layer was dried over anhydrous potassium carbonate, filtered and evaporated to dryness. The residual oil was dissolved in 400 ml of tetrahydrofuran. To the solution was added 5.75 g (0.065 mole) of unsym-dimethylethylenediamine. Tetrahydrofuran was evaporated to leave an oil, the free base of the title compound, which was reacted with oxalic acid. The oxalate salt was recrystallized from methanol-diethyl ether to give 8.86 g of the title compound (60.3%) yield as white crystals, m.p. 101.5—104°C.

Analysis:

| | | | | |
|---|---|---|---|---|
| Calculated for $C_{22}H_{31}N_3O_5S$ | : | C, 58.78; | H, 6.95; | N, 9.35 |
| Found | : | C, 58.52; | H, 6.93; | N, 9.32 |

Example 38

N'-[2-(Diethylamino)ethyl]-N-(1-methylethyl)-N-[2-[(4-methylphenyl)sulphonyl]ethyl]urea, tartrate.

To a solution of phosgene (35 ml of 12.5% in benzene) and 4.28 g (0.02 mole) Proton Sponge in 300 ml of methylene chloride was added a methylene chloride was added a methylene chloride solution of 6.64 g (0.0195 mole) 1-methyl-N-[2-[(4-methylphenyl)sulphonyl]ethyl]ethanamine (oil in Preparation 14) over a 45 minute period. The resulting solution was stirred for 2 hr at room temperature and extracted with 1N sulphuric acid. The methylene chloride solution was dried over anhydrous potassium carbonate and filtered. The filtrate was evaporated to give an oil which was dissolved in 300 ml of tetrahydrofuran. To the solution was added 4.64 g (0.04 mole) of N,N-diethylethylenediamine. After stirring for about 50 hr, the tetrahydrofuran was removed in a rotary evaporator to give an oil. The oil was partitioned between chloroform and water. Removal of chloroform gave an oil, the free base of the title compound, which was reacted with tartaric acid and the tartrate salt recrystallized from methanol-diethyl ether to give 6.14 g of the title compound (59.1%) as a yellow solid, m.p. 122—125°C.

Analysis:

| | | | | |
|---|---|---|---|---|
| Calculated for $C_{23}H_{32}N_3O_9S$ | : | C, 51.77; | H, 7.37; | N, 7.87 |
| Found | : | C, 51.44; | H, 7.35; | N, 7,79 |

Example 39

N-[2-[(3,4-Dichlorophenyl)sulphonyl]ethyl]-N'-(dimethylamino)ethyl]-N-(1-methylethyl)urea maleate [1:1].

To a solution of 6.00 g (0.037 mole) of 1,1'-carbonyldiimidazole and 2.90 g (0.033 mole) of unsym-N,N-dimethylethylenediamine in 300 ml of tetrahydrofuran which had been stirred for 2 hr at room temperature was added a solution of 7.96 g (0.027 mole) of N-[2-[(3,4-dichlorophenyl)sulphonyl]ethyl]ethanamine in 100 ml of tetrahydrofuran. The solution was heated overnight at reflux. The solvent was removed using a rotary evaporator to give an oil residue which was dissolved in chloroform. The solution was extracted with water. Evaporation of chloroform gave an oil, the free base of the title compound which was reacted with maleic acid and the resulting salt was recrystallized from methanol-diethyl ether to give 10.12 g (71.4%) of white crystalline product, m.p. 145—146°C.

Analysis:

| | | | | |
|---|---|---|---|---|
| Calculated for $C_{20}H_{29}N_3O_7SCl_2$ | : | C, 45.63; | H, 5.55; | N, 7.98 |
| Found | : | C, 45.63; | H, 5.61; | N, 8.11 |

Example 40

N'-[2-(Diethylamino)ethyl]-N-(1-methylethyl)-N-[2-[(4-methylphenyl)sulphinyl]ethyl]urea hemihydrate.

To a solution of 9.08 g (0.056 mole) of 1,1'-carbonyldiimidazole and 5.58 g (0.048 mole) of unsym-N,N-diethylethylenediamine in tetrahydrofuran which had been stirred for 2 hr at room temperature was added a solution of 9.43 g (0.419 mole) of 1-methyl-N-[2-[(4-methylphenyl)sulphinyl]ethyl]ethanamine (oil in Preparation 19) in tetrahydrofuran. The solution was heated overnight at gentle reflux. The reaction mixture was stripped to dryness and the resulting oil residue was partitioned between chloroform and water. Evaporation of the chloroform layers gave an oil which was chromatographed by slurrying with methanol-chloroform (20—80 vol %) and silica gel with repeated filtration. Filtrates were combined and solvent removed by evaporation. The residue was dried *in vacuo* overnight to give 6.98 g (44.2%) light-brown oil.

Analysis:

| | | | | |
|---|---|---|---|---|
| Calculated for $C_{38}H_{68}N_6O_5S_2$ | : | C, 60.60; | H, 9.10; | N, 11.16 |
| Found | : | C, 60.95; | H, 9.12; | N, 11.35 |

Example 41

N'-[3-(Diethylamino)propyl]-N-(1-methylethyl)-N-[3-(phenylsulphonyl)propyl]urea oxalate [1:1] hemihydrate.

To a solution of 6.17 g (0.038 mole) of 1,1'-carbonyldiimidazole and 4.28 g (0.033 mole) of unsym-N,N-diethyl-1,3-propanediamine in 300 ml of tetrahydrofuran which had stirred for 2 hours at room temperature was added a solution of 7.23 g (0.03 mole) of 1-methyl-N-[3-(phenylsulphonyl)propyl]ethanamine (oil in Preparation 26) in 100 ml of tetrahydrofuran. The resulting solution was heated overnight at reflux. The reaction mixture was stripped to dryness and the residue partitioned between chloroform and water. The chloroform layer was evaporated to give an oil, the free base of the title compound. The oil was reacted with oxalic acid and the resulting salt was recrystallized from methanol-diethyl ether to give 12.31 g (82.6%) of white crystalline product, m.p. 120—121.5°C.

Analysis:

| | | | | |
|---|---|---|---|---|
| Calculated for $C_{44}H_{76}N_6O_{15}S_2$ | : | C, 53.21; | H, 7.71; | N, 8.46 |
| Found | : | C, 53.36; | H, 7.62; | N, 8.96 |

Example 42

N'-[2-(Diethylamino)ethyl]-N-(1-methylethyl)-N-[3-(phenylsulphonyl)propyl]urea oxalate [1:1] hemihydrate.

To a solution of 9.73 g (0.06 mole) of 1,1'-carbonyldiimidazole and 6.06 g (0.054 mole) of unsym N,N-diethylethylenediamine in 350 ml of tetrahydrofuran which had stirred for 1.5 hr at room temperature was added a solution of 12.06 g (0.05 mole) 1-methyl-N-[3-(phenylsulphonylpropyl]ethanamine (oil in Preparation 26) in 100 ml of tetrahydrofuran. The resulting solution was heated overnight at gentle reflux. The reaction mixture was stripped to dryness and the residue partitioned between chloroform and water. The chloroform layer was evaporated to give an oil, the free base of the title compound which was reacted with oxalic acid and the resulting salt was recrystallized from methanol-diethyl ether to give 20.46 g (84.8%) of white crystalline product, m.p. 117—118.5C.

Analysis:

| | | | | |
|---|---|---|---|---|
| Calculated for $N_{42}H_{72}N_6O_{15}S_2$ | : | C, 52.27; | H, 7.52; | N, 8.71 |
| Found | : | C, 52.62; | H, 7.35; | N, 8.61 |

Example 43

N'-[3-(Diethylamino)propyl]-N-(1-methylethyl)-N-[2-(phenylsulphonyl)ethyl]urea·1/4 hydrate

To a solution of 6.00 g (0.037 mole) of 1,1'-carbonyldiimidazole and 4.17 g (0.032 mole) unsym-N,N-diethylethylenediamine in 300 ml of tetrahydrofuran which had stirred at room temperature for 2.5 hr was added a solution of 6.81 g (0.03 mole) of 1-methyl-N-[2-(phenylsulphonyl)ethyl]ethanamine (oil in Preparation 6) in 100 ml of tetrahydrofuran. The solution was heated overnight at reflux. The reaction mixture was stripped to dryness and the resulting oil residue was partitioned between chloroform and water. Evaporation of the chloroform layer gave an oil which was chromatographed by slurrying with 95 to 98 vol % chloroform — 2 to 5 vol % acetone and 50—90 vol % chloroform and 10—50 vol % methanol and silica gel with repeated filtration. Filtrates were combined and solvent removed by evaporation. The residual oil was triturated with diethyl ether. The light brown oil remaining after decanting the ether and drying *in vacuo* overnight at 90°C. weighed 5.29 g (44.9%).

Analysis:

| | | | | |
|---|---|---|---|---|
| Calculated for $C_{38}H_{64}N_6O_7$ | : | C, 58.13; | H, 8.78; | N, 10.70 |
| Found | : | C, 58.31; | H, 8.64; | N, 10.80 |

Example 44

N'-[2-(Diethylamino)ethyl]-N-[2-[(4-fluorophenyl)sulphonyl]ethyl]-N-(1-methylethyl)urea

To a solution of 6.49 g (0.04 mole) of 1,1-carbonyldiimidazole and 4.06 g (0.035 mole) of unsym-N,N-diethylethylenediamine in 400 ml of tetrahydrofuran which had stirred at room temperature for 1.5 hr was added 8.45 g (0.03 mole) of N-[2-[(4-fluorophenyl)sulphonyl]ethyl]-1-methylethanamine. The solution was heated overnight at reflux. The reaction mixture was stripped to dryness and partitioned 5 times between water and methylene chloride. The methylene chloride layers were combined and evaporated to dryness.

The oil was partitioned between diethyl ether and water several times. The ether layers were combined and solvent was removed by evaporation. After drying *in vacuo* at 80°C for 36 hr, 4.56 g (39.2%) of brown oil was obtained.

Analysis:

| | | | | | | | |
|---|---|---|---|---|---|---|---|
| Calculated for $C_{18}H_{30}N_3O_3SF$ | : | C, | 55.79; | H, | 7.80; | N, | 10.87 |
| Found | : | C, | 55.31; | H, | 7.80; | N, | 10.48 |

Example 45

N-[2-(Diethylamino)ethyl]-N-methyl-N′-(1-methylethyl N′-[2-(phenylsulphonyl)ethyl]urea maleage.

The title compound was prepared by Method A and the procedure of Example 1 by reacting in sequence:

phosgene, Proton Sponge

N-[2-(phenylsulphonyl)ethyl]-2-propanamine, and

N,N-diethyl-N′-methylethylenediamine to give the free base of the title compound which was then reacted with maleic acid to give the title compound.

Example 46

N-(1-Methylethyl)-N′-[2-[bis(1-methylethyl)amino]ethyl]-N-[2-(phenylsulphonyl ethyl]urea maleate.

The title compound was prepared by Method B, reacting in sequence:

1,1′-carbonyldiimidazole,

N,N-bis(1-methylethyl)-1,2-ethanediamine, and

N-[2-(phenylsulphonyl)ethyl]-2-propanamine, to give the free base of the title compound which was then reacted with maleic acid to give the title compound.

Example 47

N-(1-Methylethyl)-N′-[2-[methyl-(2-phenylethyl)amino]ethyl]-N-[2-phenylsulphonyl)ethyl]urea maleate.

The title compound was prepared by Method B, reacting in sequence:

1,1′-carbonyldiimidazole,

N-methyl-N-(2-phenylethyl)-1,2-ethanediamine, and

N-[2-(phenylsulphonyl)ethyl]-2-propanamine, to give the free base of the title compound which was then reacted with maleic acid to give the title compound.

Example 48

N-(1-Methylethyl)-N′-[2-[methyl(phenylmethyl)amino]ethyl]-N-[2-(phenylsulphonyl)ethyl]urea maleate.

The title compound was prepared by Method B, reacting in sequence:

1,1′-carbonyldiimidazole,

N-methyl-N-(phenylmethyl)-1,2-ethanediamine, and

N-[2-(phenylsulphonyl)ethyl]-2-propanamine, to give the free base of the title compound which was then reacted with maleic acid to give the title compound.

Example 49

N-[2-[(4-Bromophenyl)sulphonyl]ethyl]-N′-[2-(diethylamino)ethyl]-N-(1-methylethyl)urea maleate.

The title compound was prepared by Method B, reacting in sequence:

1,1-carbonyldiimidazole,

unsym-N,N-diethylenediamine, and

N-[2-[(4-bromophenyl)sulphonyl]ethyl]-2-propanamine, to give the free base of the title compound which was then reacted with maleic acid to give the title compound.

Example 50

N′-[2-(Diethylamino)ethyl]-N-[2-[[4-(1,1-dimethylethyl)phenyl]sulphonyl]ethyl]-N-(1-methylethyl)urea maleate.

The title compound was prepared by Method B, reacting in sequence:

1,1′-carbonyldiimidazole,

unsym-N,N-diethylethylenediamine, and

N-[2-[(4-*t*-butylphenyl)sulphonyl]ethyl]-2-propanamine, to give the free base of the title compound which was then reacted with maleic acid to give the title compound.

Example 51

N-[2-(Diethylamino)ethyl]-N′-methyl-N-(1-methylethyl)-N′-[2-(phenylsulphonyl)ethyl]urea maleate.

The title compound was prepared by Method A and the procedure of Example 1 by reacting in sequence:

phosgene, Proton Sponge,

N-methyl-2-(phenylsulfonyl)ethanamine, and

N,N-diethyl-N′-isopropylethylenediamine, to give the free base of the title compound which was then reacted with maleic acid to give the title compound.

Example 52

N′-[2-(Diethylamino)ethyl]-N-[2-[(2-furanylmethyl)thio]ethyl]-N-(1-methylethyl)urea maleate.

The title compound was prepared by Method B, reacting in sequence:

1,1′-carbonyldiimidazole,

unsym-N,N-diethylethylenediamine, and

N-[2-[(2-furanylmethyl)thio]ethyl]-2-propanamine, to give the free base of the title compound which was then reacted with maleic acid to give the title compound.

Example 53

N′-[2-(Diethylamino)ethyl]-N-[2-[(2-furanylmethyl)sulphonyl]ethyl]-N-(1-methylethyl)urea

The title compound was prepared by Method B, reacting in sequence:

1,1′-carbonyldiimidazole,

unsym-N,N-diethylethylenediamine, and

N-[2-[(2-furanylmethyl)sulphonyl]ethyl]-2-propanamine, to give the ree base of the title compound which was then reacted with maleic acid to give the title compound.

Example 54

N′-[2-(Diethylamino)ethyl]-N-[2-[(2-furanylmethyl)sulphinyl]ethyl]-N-(1-methylethyl)urea maleate.

The title compound was prepared by Method B, reacting in sequence:

1,1′-carbonyldiimidazole,

unsym-N,N-diethylethylenediamine, and

N-[2-[(2-furanylmethyl)sulphinyl]ethyl]-2-propanamine, to give the free base of the title compound which was then reacted with maleic acid to give the title compound.

Example 55

N′-[2-(Diethylamino)ethyl]-N-(1-methylethyl)-N-[2-[(phenylmethyl)sulphonyl]ethyl]urea maleate.

The title compound was prepared by Method B, reacting in sequence:

1,1′-carbonyldiimidazole,

unsym-N,N-diethylethylenediamine, and

N-[2-[(phenylmethyl)sulphonyl]ethyl]-2-propanamine, to give the free base of the title compound which was then reacted with maleic acid to give the title compound.

Example 56

N′-[2-(Diethylamino)ethyl]-N-(1-methylethyl)-N-[2-[[3-(trifluoromethyl)phenyl]sulphonyl]ethyl]urea maleate.

The title compound was prepared by Method B, reacting in sequence:

1,1′-carbonyldiimidazole,

unsym-N,N-diethylethylenediamine, and

N-[2-[[3-(trifluoromethyl)phenyl]sulphonyl]ethyl]-2-propanamine, to give the free base of the title compound which was then reacted with maleic acid to give the title compound.

Example 57

N′-[2-(Diethylamino)ethyl]-N-methyl-N-[2-(phenyl-sulphonyl)ethyl]urea maleate.

The title compound was prepared by Method B, reacting in sequence:

1,1′-carbonyldiimidazole,

unsym-N,N-diethylethylenediamine, and

N-methyl-2-(phenylsulphonyl)ethanamine, to give the free base of the title compound which was then reacted with maleic acid to give the title compound.

Example 58

N′-[2-(Diethylamino)ethyl]-N-(1-methylethyl)-N-[2-[(phenylmethyl)thio]ethyl]urea maleate.

The title compound was prepared by Method B, reacting in sequence:

1,1′-carbonyldiimidazole,

unsym-N,N-diethylethylenediamine, and

N-[2-[(phenylmethyl)thio]ethyl]-2-propanamine, to give the free base of the title compound which was then reacted with maleic acid to give the title compound.

Example 59

N′-[2-(Diethylamino)ethyl]-N-(1-methylethyl)-N-[2-[(phenylmethyl)sulphinyl]ethyl]urea maleate.

The title compound was prepared by Method B, reacting in sequence:

1,1′-carbonyldiimidazole,

unsym-N,N-diethylethylenediamine, and

N-[2[(phenylmethyl)sulphinyl]ethyl]-2-propanamine, to give the free base of the title compound which was then reacted with maleic acid to give the title compound.

Example 60

N'-[2-(Diethylamino)ethyl]-N'-phenyl-N-[2-(phenylsulphonyl)ethyl]urea maleate.

The title compound was prepared by Method C, reacting in sequence:

1,1'-carbonyldiimidazole,

2-(phenylsulphonyl)ethanamine, and

N,N-diethyl-N'-phenylethylenediamine, to give the free base of the title compound which was then reacted with maleic acid to give the title compound.

Tables 1A and 1B both refer to the compound of Formula I.

TABLE 1A

| Ex. No. | Ar | B | alk[1] | R[1] | X | R[2] |
|---|---|---|---|---|---|---|
| 1 | $4-Cl-C_6H_4$ | –S– | $-(CH_2)_2-$ | $-CH(CH_3)_2$ | O | H |
| 2 | $2-C_{10}H_7-$ | –S– | $-(CH_2)_2-$ | $-CH(CH_3)_2$ | O | H |
| 3 | $4-Cl-C_6H_4-$ | $-S(O)_2-$ | $-(CH_2)_2-$ | $-CH(CH_3)_2$ | O | H |
| 4 | $4-Cl-C_6H_4-$ | $-S(O)_2-$ | $-(CH_2)_2-$ | $-CH(CH_3)_2$ | O | H |
| 5 | $4-Cl-C_6H_4-$ | $-S(O)_2-$ | $-(CH_2)_2-$ | H | O | $-CH(CH_3)_2$ |
| 6 | $4-Cl-C_6H_4-$ | $-S(O)_2-$ | $-(CH_2)_2-$ | $-CH(CH_3)_2$ | S | H |
| 7 | $4-Cl-C_6H_4-$ | $-S(O)-$ | $-(CH_2)_2-$ | $-CH(CH_3)_2$ | O | H |
| 8 | $C_6H_5-$ | $-S(O)_2-$ | $-(CH_2)_2-$ | $-CH(CH_3)_2$ | O | H |
| 9 | $C_6H_5-$ | $-S(O)_2-$ | $-(CH_2)_2-$ | $-CH(CH_3)_2$ | O | H |
| 10 | $l-C_{10}H_7-$ | $-S(O)_2-$ | $-(CH_2)_2-$ | $-CH(CH_3)_2$ | O | H |
| 11 | inden–4–yl– | $-S(O)_2-$ | $-(CH_2)_2-$ | $-CH(CH_3)_2$ | O | H |
| 12 | inden–5–yl– | $-S(O)_2-$ | $-(CH_2)_2-$ | $-CH(CH_3)_2$ | O | H |
| 13 | $4-CH_3-C_6H_4-$ | $-S(O)_2-$ | $-(CH_2)_2-$ | $-CH(CH_3)_2$ | O | H |
| 14 | $4-(OCH_3)-C_6H_4-$ | $-S(O)_2-$ | $-(CH_2)_2-$ | $-CH(CH_3)_2$ | O | H |
| 15 | $3,5-Cl_2-C_6H_3-$ | $-S(O)_2-$ | $-(CH_2)_2-$ | $-CH(CH_3)_2$ | O | H |
| 16 | $3,4,5-(OCH_3)_3-C_6H_2-$ | $-S(O)_2-$ | $-(CH_2)_2-$ | $-CH(CH_3)_2$ | O | H |
| 17 | $4-F-C_6H_4-$ | $-S(O)_2-$ | $-(CH_2)_2-$ | $-CH(CH_3)_2$ | O | H |
| 18 | $4-CF_3-C_6H_4-$ | $-S(O)_2-$ | $-(CH_2)_2-$ | $-CH(CH_3)_2$ | O | H |
| 19 | $4-CN-C_6H_4-$ | $-S(O)_2-$ | $-(CH_2)_2-$ | $-CH(CH_3)_2$ | O | H |
| 20 | $4-Cl-C_6H_4-$ | $-S(O)_2-$ | $-(CH_2)_3-$ | $-CH(CH_3)_2$ | O | H |
| 21 | $C_6H_5-$ | $-S(O)_2-$ | $-(CH_2)_3-$ | H | O | $-CH(CH_3)_2$ |
| 22 | $C_6H_5-$ | $-S(O)_2-$ | $-(CH_2)_2-$ | $-CH(CH_3)_2$ | O | H |
| 23 | $C_6H_5-$ | $-S(O)_2-$ | $-(CH_2)_2-$ | $-CH(CH_3)_2$ | O | H |
| 24 | $C_6H_5-$ | $-S(O)_2-$ | $-(CH_2)_2-$ | $-CH(CH_3)_2$ | O | H |
| | $C_6H_5$ | $-S(O)_2-$ | $-(CH_2)_2-$ | $-CH(CH_3)_2$ | O | H |
| | $C_6H_5-$ | $-S(O)_2-$ | $-(CH_2)_2-$ | $-CH(CH_3)_2$ | O | H |

TABLE 1A (Continued)

| Ex. No. | Ar | B | alk[1] | $R^1$ | X | $R^2$ |
|---|---|---|---|---|---|---|
| 25 | $C_6H_5-$ | $-S(O)_2-$ | $-(CH_2)_2-$ | $-C_6H_{11}$ | O | H |
| 26 | $C_6H_5-$ | $-S(O)_2-$ | $-(CH_2)_2-$ | $-C_6H_5$ | O | H |
| 27 | $C_6H_5-$ | $-S(O)_2-$ | $-(CH_2)_2-$ | $-CH_2-C_6H_5$ | O | H |
| 28 | $C_6H_5-$ | $-S(O)_2-$ | $-(CH_2)_2-$ | $-CH(CH_3)_2$ | O | H |
| 29 | $C_6H_5-$ | $-S(O)_2-$ | $-(CH_2)_2-$ | $-CH(CH_3)_2$ | O | $-CH_3$ |
| 30 | $C_6H_5-$ | $-S(O)_2-$ | $-(CH_2)_2-$ | $-CH(CH_3)_2$ | S | H |
| 31 | $4-CH_3-C_6H_4$ | $-S(O)_2-$ | $-(CH_2)_2-$ | $-CH(CH_3)_2$ | O | H |
| 32 | $4-Cl-C_6H_4$ | $-S(O)_2-$ | $-(CH_2)_2-$ | $-CH(CH_3)_2$ | O | H |
| 33 | $4-Cl-C_6H_4$ | $-S(O)_2-$ | $-(CH_2)_3-$ | H | O | $-CH(CH_3)_2$ |
| 34 | $1-C_{10}H_7$ | $-S-$ | $-(CH_2)_2-$ | $-CH(CH_3)_2$ | O | H |
| 35 | $1-C_{10}H_7$ | $-S(O)-$ | $-(CH_2)_2-$ | $-CH(CH_3)_2$ | O | H |
| 36 | $4-NO_2-C_6H_4$ | $-S(O)_2-$ | $-(CH_2)_2-$ | $-CH(CH_3)_2$ | O | H |
| 37 | $1-C_{10}H_7$ | $-S-$ | $-(CH_2)_2-$ | $-CH(CH_3)_2$ | O | H |
| 38 | $4-CH_3-C_6H_4$ | $-S(O)_2-$ | $-(CH_2)_2-$ | $-CH(CH_3)_2$ | O | H |
| 39 | $3,4-(Cl_2)-C_6H_3-$ | $-S(O)_2-$ | $-(CH_2)_2-$ | $-CH(CH_3)_2$ | O | H |
| 40 | $4-CH_3-C_6H_4-$ | $-S(O)-$ | $-(CH_2)_2-$ | $-CH(CH_3)_2$ | O | H |
| 41 | $C_6H_5$ | $-S(O)_2-$ | $-(CH_2)_3-$ | $-CH(CH_3)_2$ | O | H |
| 42 | $C_6H_5-$ | $-S(O)_2-$ | $-(CH_2)_3-$ | $-CH(CH_3)_2$ | O | H |
| 43 | $C_6H_5-$ | $-S(O)_2-$ | $-(CH_2)_2-$ | $-CH(CH_3)_2$ | O | H |
| 44 | $4-F-C_6H_4-$ | $-S(O)_2-$ | $-(CH_2)_2-$ | $-CH(CH_3)_2$ | O | H |
| 45 | $C_6H_5-$ | $-S(O)_2-$ | $-(CH_2)_2-$ | $-CH(CH_3)_2$ | O | $-CH_3$ |
| 46 | $C_6H_5-$ | $-S(O)_2-$ | $-(CH_2)_2-$ | $-CH(CH_3)_2$ | O | H |
| 47 | $C_6H_5-$ | $-S(O)_2-$ | $-(CH_2)_2-$ | $-CH(CH_3)_2$ | O | H |
| 48 | $C_6H_5-$ | $-S(O)_2-$ | $-(CH_2)_2-$ | $-CH(CH_3)_2$ | O | H |
| 49 | $4-Br-C_6H_4-$ | $-S(O)_2-$ | $-(CH_2)_2-$ | $-CH(CH_3)_2$ | O | H |
| 50 | $4-t-Bu-C_6H_4-$ | $-S(O)_2-$ | $-(CH_2)_2-$ | $-CH(CH_3)_2$ | O | H |
| 51 | $C_6H_5-$ | $-S(O)_2-$ | $-(CH_2)_2-$ | $-CH_3$ | O | $-CH(CH_3)_2$ |
| 52 | 2-furanyl$-CH_2-$ | $-S-$ | $-(CH_2)_2-$ | $-CH(CH_3)_2$ | O | H |
| 53 | 2-furanyl$-CH_2-$ | $-S(O)_2-$ | $-(CH_2)_2-$ | $-CH(CH_3)_2$ | O | H |

TABLE 1A (Continued)

| Ex. No. | Ar | B | $alk^1$ | $R^1$ | X | $R^2$ |
|---|---|---|---|---|---|---|
| 54 | 2-furanyl–$CH_2$– | –S(O)– | –$(CH_2)_2$– | –$CH(CH_3)_2$ | O | H |
| 55 | $C_6H_5$–$CH_2$– | –$S(O)_2$– | –$(CH_2)_2$– | –$CH(CH_3)_2$ | O | H |
| 56 | 3–$CF_3$–$C_6H_4$– | –$S(O)_2$– | –$(CH_2)_2$– | –$CH(CH_3)_2$ | O | H |
| 57 | $C_6H_5$– | –$S(O)_2$– | –$(CH_2)_2$– | –$CH_3$ | O | H |
| 58 | $C_6H_5$–$CH_2$– | –S– | –$(CH_2)_2$– | –$CH(CH_3)_2$ | O | H |
| 59 | $C_6H_5$–$CH_2$ | –S(O)– | –$(CH_2)_2$– | –$CH(CH_3)_2$ | O | H |
| 60 | $C_6H_5$ | –$S(O)_2$– | –$(CH_2)_2$– | H | O | $C_6H_5$– |

TABLE 1B

| Ex. No. | $alk^2$ | –$NR^3R^4$ | Salt | M.P., °C |
|---|---|---|---|---|
| 1 | –$(CH_2)_2$– | –$N(CH_3)_2$ | 1.5 fumarate | 101–103.5 |
| 2 | –$(CH_2)_2$– | –$N(CH_3)_2$ | 1.5 succinate | 96–98 |
| 3 | –$(CH_2)_2$– | –$N(CH_3)_2$ | maleate | 134–135 |
| 4 | –$(CH_2)_2$– | –$N(C_2H_5)_2$ | tartrate | 135–137 |
| 5 | –$(CH_2)_2$– | –$N(CH_3)_2$ | maleate | 125–126.5 |
| 6 | –$(CH_2)_2$– | –$N(CH_3)_2$ | 0.5 fumarate | 141–142 |
| 7 | –$(CH_2)_2$– | –$N(CH_3)_2$ | fumarate | 112.5–114.5 |
| 8 | –$(CH_2)_2$– | –$N(CH_3)_2$ | maleate | 103–105 |
| 9 | –$(CH_2)_2$– | –$N(C_2H_5)_2$ | maleate | 88–90 |
| 10 | –$(CH_2)_2$– | –$N(CH_3)_2$ | maleate | 126–128 |
| 11 | –$(CH_2)_2$– | –$N(C_2H_5)_2$ | maleate | – |
| 12 | –$(CH_2)_2$– | –$N(C_2H_5)_2$ | maleate | – |
| 13 | –$(CH_2)_2$– | –$N(C_2H_5)_2$ | maleate | – |
| 14 | –$(CH_2)_2$– | –$N(C_2H_5)_2$ | maleate | – |
| 15 | –$(CH_2)_2$– | –$N(C_2H_5)_2$ | maleate | – |
| 16 | –$(CH_2)_2$– | –$N(C_2H_5)_2$ | maleate | – |
| 17 | –$(CH_2)_2$– | –$N(CH_3)_2$ | maleate | – |
| 18 | –$(CH_2)_2$– | –$N(C_2H_5)_2$ | maleate | – |
| 19 | –$(CH_2)_2$– | –$N(C_2H_5)_2$ | maleate | – |

TABLE 1B (Continued)

| Ex. No. | $alk^2$ | $-NR^3R^4$ | Salt | M.P., °C |
|---|---|---|---|---|
| 20 | $-(CH_2)_2-$ | $-N(C_2H_5)_2$ | maleate | – |
| 21 | $-(CH_2)_2-$ | $-N(C_2H_5)_2$ | maleate | – |
| 22 | $-(CH_2)_3-$ | $-N(CH_3)_2$ | maleate | – |
| 23 | $-(CH_2)_2-$ | 1-pyrrolinyl | oxalate | – |
| 24 | $-(CH_2)_2-$ | 1-piperidinyl | oxalate | – |
| | $-(CH_2)_2-$ | 4-morpholinyl | oxalate | – |
| | $-(CH_2)_2-$ | 4-methyl-piperazin-1-yl | oxalate | – |
| 25 | $-(CH_2)_2-$ | $-N(C_2H_5)_2$ | maleate | – |
| 26 | $-(CH_2)_2-$ | $-N(C_2H_5)_2$ | maleate | – |
| 27 | $-(CH_2)_2-$ | $-N(C_2H_5)_2$ | maleate | – |
| 28 | $-(CH_2)_2-$ | $-NH_2$ | – | – |
| 29 | $-(CH_2)_2-$ | $-NHCH_3$ | maleate | – |
| 30 | $-(CH_2)_2-$ | $-N(C_2H_5)_2$ | fumarate | – |
| 31 | $-(CH_2)_2-$ | $-N(CH_3)_2$ | maleate | – |
| 32 | $-(CH_2)_2-$ | $-N(CH_3)_2$ | maleate | – |
| 33 | $-(CH_2)_2-$ | $-N(CH_3)_2$ | maleate | – |
| 34 | $-(CH_2)_2-$ | $-N(CH_3)_2$ | maleate | – |
| 35 | $-(CH_2)_2-$ | $-N(CH_3)_2$ | maleate | – |
| 36 | $-(CH_2)_2-$ | $-N(CH_3)_2$ | maleate | – |
| 37 | $-(CH_2)_2-$ | $-N(CH_3)_2$ | oxalate | 101.5–104 |
| 38 | $-(CH_2)_2-$ | $-N(C_2H_5)_2$ | tartrate | 122–125 |
| 39 | $-(CH_2)_2-$ | $-N(CH_3)_2$ | maleate | 145–146 |
| 40 | $-(CH_2)_2-$ | $-N(C_2H_5)_2$ | 0.5 $H_2O$ | oil |
| 41 | $-(CH_2)_3-$ | $-N(C_2H_5)_2$ | oxalate 0.5 $H_2O$ | 120–121.5 |
| 42 | $-(CH_2)_2-$ | $-N(C_2H_5)_2$ | oxalate 0.5 $H_2O$ | 117–118.5 |
| 43 | $-(CH_2)_3-$ | $-N(C_2H_5)_2$ | 1/4 $H_2O$ | oil |

TABLE 1B (Continued)

| Ex. No. | $alk^2$ | $-NR^3R^4$ | Salt | M.P., °C |
|---|---|---|---|---|
| 44 | $-(CH_2)_2-$ | $-N(C_2H_5)_2$ | – | oil |
| 45 | $-(CH_2)_2-$ | $-N(C_2H_5)_2$ | maleate | – |
| 46 | $-(CH_2)_2-$ | $-N-CH(CH_3)_2$ 2 | maleate | – |
| 47 | $-(CH_2)_2-$ | $-N(CH_3)$ $-(CH)_2-\phi$ | maleate | – |
| 48 | $-(CH_2)_2-$ | $-N(CH_3)$ $(-CH_2\phi)$ | maleate | – |
| 49 | $-(CH_2)_2-$ | $-N(C_2H_5)_2$ | maleate | – |
| 50 | $-(CH_2)_2-$ | $-N(C_2H_5)_2$ | maleate | – |
| 51 | $-(CH_2)_2-$ | $-N(C_2H_5)_2$ | maleate | – |
| 52 | $-(CH_2)_2-$ | $-N(C_2H_5)_2$ | maleate | – |
| 53 | $-(CH_2)_2-$ | $-N(C_2H_5)_2$ | maleate | – |
| 54 | $-(CH_2)_2-$ | $-N(C_2H_5)_2$ | maleate | – |
| 55 | $-(CH_2)_2-$ | $-N(C_2H_5)_2$ | maleate | – |
| 56 | $-(CH_2)_2-$ | $-N(C_2H_5)_2$ | maleate | – |
| 57 | $-(CH_2)_2-$ | $-N(C_2H_5)_2$ | maleate | – |
| 58 | $-(CH_2)_2-$ | $-N(C_2H_5)_2$ | maleate | – |
| 59 | $-(CH_2)_2-$ | $-N(C_2H_5)_2$ | maleate | – |
| 60 | $-(CH_2)_2-$ | $-N(C_2H_5)_2$ | maleate | – |

## Pharmacology

The action of compounds of this invention is correcting cardiac arrhythmias or preventing cardiac arrhythmias was demonstrated by the following procedures:

### Ouabain Induced Arrhythmias

Correction of existing cardiac arrhythmias of ventricular origin was carried out on (1) adult mongrel dogs which were under barbiturate anesthesia during the test. A Grass Model 7 polygraph was used for recording femoral arterial blood pressure (Statham P23AC transducer) and the electrocardiogram (Grass 7P4 preamplifier). Ouabain was given intravenously in an initial dose of 40 µg/kg and in a second dose of 20 µg/kg 30 minutes after the first dose and in subsequent doses of 10 µg/kg which were repeated at 15 min. intervals as required for producing cardiac arrhythmias that persisted for at least 15 minutes. When the arrhythmias were established, the test compounds were administered by infusion (Harvard Model 942 Infusion Pump) into a femoral vein at a rate of 1 mg/kg/min. Concentration of compound was adjusted according to the weight of the dog to allow a volume infusion of 1 ml/min. The compound was considered to be active as an antiarrhythmic agent if reversion to the sinus rhythm occurred and this was maintained for at least 30 min.

Coronary Artery Ligation Induced Arrhythmias

Adult mongrel dogs which were in the concious state were used for the test and cardiac arrhythmias were induced by prior (22-24 hr) surgical preparation in which blood flow through a coronary artery was occluded by use of a constrictor device as reported by Smith et al, 1973. A Grass Model 79 polygraph was used for recording the electrocardiogram (Grass 7P4 preamplifier).

The test compound was administered by infusion (Harvard Model 942 Infusion Pump) into a sapheneous vein to one group of dogs at a rate of 0.5 mg/kg/min. Concentration of compound was adjusted according to the weight of the dog to allow a volume of infusion of 0.5 ml/min. The test compound was administered orally by gavage to another group of dogs at dose levels of 10 to 40 mg/kg. The test compound was prepared in distilled water to give a total volume of 20 ml. Following the administration of the test compound, the heart rate, number of ectopic cardiac beats per min, and the percent ectopic beats (ectopic beats/HR X100) were recorded at 15 min. intervals. The compound was considered active if it abolished the ectopic ventricular frequency and caused a return to normal sinus rhythm within 2 hours of administration.

Date obtained for one preferred compound; namely, N'-[2-(diethylamino)ethyl]-N-(1-methylethyl) - N-[2-(phenylsulphonyl)ethyl]urea as represented by its maleate salt of Example 9 are shown in Table 1. The other compounds of this invention show qualitatively similar effects in one or more types of arrhythmias as represented by the foregoing tests. In general the compounds of this invention exhibit less CNS side effects than quinidine or lidocaine, the sulphones being superior in this respect and at the same time exhibiting excellent antiarrhythmic activity.

Table 1
Effect of Compound of Example 9: N'-[2-(diethylamino)ethyl]-N-(1-methylethyl)-N-[2-phenylsulphonyl)ethyl urea on Cardiac Arrhythmias in Dogs

| Arrhythmia Model | Correcting Dose Range mg/kg I.V. |
|---|---|
| Ouabain-Induced[1] | 4-15 |
| Coronary Artery Ligation Induced[2] | 2-5 |

[1]Cardiac arrhythmias produced by method of Lucchessi and Hardman, 1961, U. Pharmacol. Exp. Ther. *132,* 372-381.

[2]Cardiac arrhythmias produced by modification of method of Harris, 1950, Circulation *1,* 1318, as reported by Smith et al, 1973, Pharmacologist *15,* 192.

Pharmaceutical Compositions

The invention further provides pharmaceutical compositions for administration to a living animal body comprising, as active ingredients, at least one of the compounds according to the invention in association with a pharmaceutical carrier or excipient. The compounds are thus presented in a therapeutic composition suitable for oral, rectal, parenteral or intracardial administration. Thus, for example, compositions for oral administration are preferably solids and can take the form of capsules, tablets or coated tablets containing carriers conveniently used in the pharmaceutical art. Suitable tableting excipients include lactose, potato and maize starches, talc, gelatin and stearic and silicic acids, magnesium stearate and polyvinyl pyrrolidone.

For parenteral administration, the carrier or excipient can be a sterile, parenterally acceptable liquid; e.g., water, or a parenterally acceptable oil; e.g., arachis oil, contained in ampoules.

In compositions for rectal administration the carrier can comprise a suppository base; e.g., cocoa butter, or a glyceride.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount; i.e., such that a suitable effective dosage will be obtained consistent with the dosage form employed. The exact individual dosages, as well as daily dosages, will, of course, be determined according to standard medical principles under the direction of a physician or veterinarian. Generally, the pharmacology on animals suggests that the oral dosage effective to correct arrhythmias will be about 3 times that of the intravenous dosage. The animal data also suggest dosage requirements will be about half that of quinidine for the more active compounds.

Based on the animal data, allowing for variation in species and severity of cardiac arrhythmias unit dosages containing an amount of compound equivalent to about 1 to about 100 mg/kg of body weight, are contemplated. Based on all of the above considerations, a choice in a range of unit oral dosages for humans of about 10 to about 1000 mg is contemplated, preferably about 10 to 600 mg for a more active

compound such as that of Example 3A and B. Daily dosages of about 30 to 2400 mg are contemplated for humans and obviously several unit dosage forms may be administered at about the same time. However, the scope of the invention is not to be limited by these contemplations due to the uncertainty in transpositions discussed above.

Examples of unit dosage compositions are as follows:

Example 61 — Capsules

| Ingredients | Per Capsule |
|---|---|
| 1. Active ingredient | 10.0 mg |
| 2. Lactose | 146.0 mg |
| 3. Magnesium Stearate | 4.0 mg |

Ingredients 1, 2 and 3 were first blended. This blend was then milled, blended again and this milled blend was then filled into #1 hard gelatin capsules.

Example 62

10 mg tablets were prepared as follows:

| Ingredients | Mg/Tablet |
|---|---|
| 1. Active ingredient | 10.0 mg |
| 2. Corn starch | 20.0 mg |
| 3. Kelacid | 20.0 mg |
| 4. Keltose | 20.0 mg |
| 5. Magnesium Stearate | 1.3 mg |

Ingredients 1, 2, 3 and 4 were blended together. Sufficient water was added portionwise to this blend with careful stirring after each addition. Such additions of water and stirring were continued until the mass was of a consistency to permit its conversion to wet granules. The wet mass was converted to granules by passing it through the oscillating granulator, using an 8-mesh screen (U.S. Sieve series which has openings of 2.38 mms). The wet granules were then dried in an oven at 140°F (60°C). The dried granules were then passed through an oscillating granulator, using a 10-mesh screen (U.S. Sieve series which has openings of 1.68 mms). The dry granules were then lubricated with 0.5% magnesium stearate. The lubricated granules were compressed on a suitable tablet press to form tablets.

Example 63

50 mg tablets were prepared following the procedure of Example 61 using the following ingredients:

| Ingredients | Mg/Tablet |
|---|---|
| 1. Active ingredient | 50.0 mg |
| 2. Milo starch | 20.0 mg |
| 3. Corn starch | 38.0 mg |
| 4. Lactose | 90.0 mg |
| 5. Calcium Stearate | 2.0 mg |
| | 200.0 mg |

Example 64
Intravenous Injection

| Ingredients | Per ml |
|---|---|
| 1. Active ingredient | 1.0 mg |
| 2. pH 4.0 Buffer solution | q.s. to 1.0 ml |

The active ingredient was dissolved in the buffer solution. This solution was aseptically filtered. The sterile solution was aseptically filled into sterile ampoules. The ampoules were sealed under aseptic conditions.

Example 65
Intramuscular Injection

| Ingredients | Per ml |
|---|---|
| 1. Active ingredients | 5.0 mg |
| 2. Isotonic Buffer solution | q.s. to 1.0 ml |

The active ingredient was dissolved in the buffer solution. This solution was aseptically filtered. The sterile solution was aseptically filled into sterile ampoules. The ampoules were sealed under aspectic conditions.

Example 65
Suppositories

| Ingredients | Per Suppository |
|---|---|
| 1. Active ingredient | 10.0 mg |
| 2. Polyethylene Glycol 1000 | 1350.0 mg |
| 3. Polyethylene Glycol 4000 | 450.0 mg |

Ingredients 2 and 3 were melted together and stirred until uniform. Ingredient 1 was dissolved in this molten mass and the mixture stirred until uniform. This molten mass was then poured into suppository moulds and chilled. The suppositories were removed from the moulds and wrapped.

Therapeutic compositions having cardiac arrhythmia inhibiting activity in dosage unit form, comprising a pharmaceutical carrier and a cardiac arrhythmia inhibiting amount of a compound of Formula I or a pharmaceutically acceptable acid addition salt thereof are therefore an embodiment of this invention.

**Claims for the Contracting States: BE CH DE FR GB IT LI LU NL SE**

1. A compound having the formula:

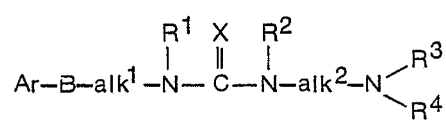

wherein:

Ar represents a 1 or 2-naphthyl, 2,3-dihydro-1H-inden-4(or 5)yl, 2-furanyl, phenyl or substituted phenyl group the said substituted phenyl group being substituted by 1—3 radicals which may be the same or different which radicals are selected from loweralkyl, loweralkoxy, halogen, trifluoromethyl, nitro, cyano, or

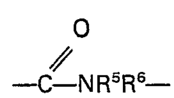

groups, wherein $R^5$ and $R^6$ each represents a hydrogen atom or a loweralkyl group, and Ar may include one intervening methylene group attached to B,

$R^1$ and $R^2$ each represent a hydrogen atom or a loweralkyl, cycloalkyl, phenyl or phenyl-loweralkyl group wherein phenyl may be substituted by a halogen atom or a loweralkyl or loweralkoxy group,

X represents an oxygen or sulphur atom,
B represents

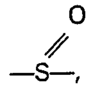, —S— or 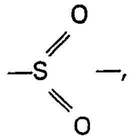,

$R^3$ and $R^4$ each represent a hydrogen atom or a loweralkyl, phenyl or phenyl-loweralkyl group wherein phenyl may be substituted by a halogen atom or a loweralkyl or loweralkoxy group and may be the same or different, or $R^3$ and $R^4$ taken together with the adjacent nitrogen may form a heterocyclic residue,

$alk^1$ and $alk^2$ each represent a loweralkylene or loweralkylene-loweralkyl group and may be the same or different, and

the pharmaceutically acceptable addition salts and hydrates thereof.

2. A compound of Claim 1 which is N-[2-[(4-chlorophenyl)thio]ethyl] - N′ - [2-(dimethylamino)ethyl] - N - (1-methylethyl)urea, or N-[2-[(4-chlorophenyl)thio]ethyl] - N′ - [2-(dimethylamino)ethyl] - N - (1-methylethyl) urea fumarate [1:1.5].

3. A compound of Claim 1 which is N′-[2-(dimethylamino)ethyl] - N - (1-methylethyl) - N - [2-[(2-naphthylene)thio]ethyl]urea, or N′-[2-(dimethylamino)ethyl] - N - (1-methylethyl) - N - [2-[2-naphthylene)thio]ethyl]urea, succinate [1:1.5], or N′-[2-(dimethylamino)ethyl] - N - (1-methylethyl) - N - [2-(1-naphthalenylthio)ethyl]urea, or N′-[2-(dimethylamino) ethyl] - N - (1-methylethyl) - N - [2-(1-naphthalenylthio)ethyl]urea, oxalate.

4. A compound of Claim 1 which is N-[2-[(4-chlorophenyl)sulphonyl)ethyl] - N′ - [2-(dimethylamino)ethyl] - N - (1-methylethyl)urea, or N-[2-[(4-chlorophenyl)sulphonyl)ethyl] - N′ - [2-(dimethylamino)ethyl] - N - (1-methylethyl)urea maleate [1:1], or N-[2-[(4-chlorophenyl)sulphonyl ethyl]-N′-[2-(diethylamino)ethyl] - N - (1-methylethyl)urea, or N-[2-[(4-chlorophenyl)sulphonyl]ethyl] - N′ - [2-(diethylamino)ethyl] - N - (1-methylethyl)urea, tartrate [1:1], or N-[2-[(4-chlorophenyl)sulphonyl]ethyl] - N′ - [2-(dimethylamino)ethyl] - N′ - (1-methylethyl)urea, or N-[2-[(4-chlorophenyl)sulphonyl]ethyl] - N′ - [2-(dimethylamino)ethyl] - N′ - (1-methylethyl)urea, maleate [1:1], or N-[2-[(4-chlorophenyl)sulphonyl]-ethyl] - N′ - [(dimethylamino)ethyl] - N - (1-methylethyl)thiourea, or N-[2-[(4-chlorophenyl)sulphonyl]-ethyl] - N′ - [2-(dimethylamino)ethyl] - N - (1-methylethyl) thiourea, hemifumarate.

5. A compound of Claim 1 which is N′-[2-(dimethylamino)ethyl] - N - (1-methylethyl) - N - [2-(phenylsulphonyl)ethyl]urea, or N′-[2-(dimethylamino)ethyl] - N - (1-methylethyl) - N - [2-(phenylsulphonyl)-ethyl]urea, maleate [1:1], or N′-[2-(diethylamino)ethyl] - N - (1-methylethyl) - N - [2-phenylsulphonyl)-ethyl]urea, or N′-[2-(diethylamino)ethyl] - N - (1-methylethyl) - N - [2-(phenylsulphonyl)ethyl]urea, maleate [1:1], or N′ - [3 - (diethylamino)propyl] - N - (1-methylethyl) - N - [3 - (phenylsulphonyl)propyl]urea, or N′ - [3 - (diethylamino)propyl] - N - (1 - methylethyl) - N - [3 - (phenylsulphonyl)propyl]urea, oxalate [1:1] hemihydrate, or N′ - [2 - (diethylamino)ethyl] - N - (1 - methylethyl) - N - [3 - (phenylsulphonyl)propyl] urea, or N′-[2-(diethylamino)ethyl]-N-(1-methylethyl) - N - [3-(phenylsulphonyl)propyl]urea oxalate [1:1] hemihydrate, or N′-[3-(diethylamino)propyl] - N - (1-methylethyl) - N - [2-(phenylsulphonyl)ethyl]urea, or N′-[3-(diethylamino)propyl] - N - (1-methylethyl) - N - [2-(phenylsulphonyl)ethyl]urea. 1/4 hydrate, or N′-[2-(diethylamino)ethyl] - N - [2-[(4-fluorophenyl)-sulphonyl]ethyl] - N - (1-methylethyl)urea.

6. A compound of Claim 1 which is N′-[2-(dimethylamino)ethyl] - N - (1-methylethyl) - N - [2-[(1-naphthalenesulphonyl)]ethyl]urea, or N′-[2-(dimethylamino)ethyl] - N - (1-methylethyl) - N - [2-[(1-naphthalenesulphonyl)ethyl]urea, maleate.

7. A compound of Claim 1 which is N′-[2-(diethylamino)ethyl] - N - (1-methylethyl) - N - [2-[(4-methylphenyl)sulphonyl]ethyl]urea, or N′-[2-(diethylamino)ethyl] - N - (1-methylethyl) - N - [2-[(4-methylphenyl)sulphonyl]ethyl] urea, tartrate, or N-[2-[(3,4-dichlorophenyl)sulphonyl]ethyl] - N′ - (dimethylamino)ethyl] - N - (1-methylethyl)urea, or N-[2-[(3,4-dichlorophenyl)sulphonyl]ethyl] - N′ - (dimethylamino)ethyl] - N - (1-methylethyl)urea maleate.

8. A compound of Claim 1 which is N-[2-[(4-chlorophenyl)sulphinyl]ethyl] - N′ - [2-(dimethylamino)-ethyl] - N - (1-methylethyl)urea, or N-[2-[(4-chlorophenyl)sulphinyl]ethyl] - N′ - [2-(dimethylamino)-ethyl] - N - (1-methylethyl)urea fumarate [1:1], or N′-[2-(diethylamino)ethyl] - N - (1-methylethyl) - N - [2-[(4-methylphenyl)sulphinyl]ethyl]urea, or N′-[2-(diethylamino)ethyl] - N - (1-methylethyl) - N - [2-[(4-methylphenyl)sulfinyl]ethyl]urea hemihydrate.

9. A compound as claimed in any one of Claims 1 to 8 for use in treating cardiac arrhythmias.

10. A therapeutic composition for the treatment of cardiac arrhythmias comprising (a) an effective amount of a compound as claimed in any one of Claims 1 to 9, and (b) a pharmaceutically acceptable carrier therefor.

**Claims for the Contracting State: AT**

1. A process for the preparation of a compound having the formula:

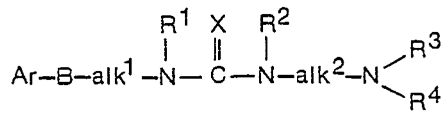

wherein:

Ar represents a 1 or 2-naphthyl, 2,3-dihydro-1H-inden-4(or 5)yl, 2-furanyl, phenyl or substituted phenyl group the said substituted phenyl group being substituted by 1—3 radicals which may be the same or different which radicals are selected from loweralkyl, loweralkoxy, halogen, trifluoromethyl, nitro, cyano, or

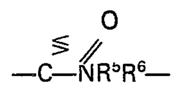

groups, wherein $R^5$ and $R^6$ each represents a hydrogen atom or a loweralkyl group, and Ar may include one intervening methylene group attached to B,

$R^1$ and $R^2$ each represent a hydrogen atom or a loweralkyl, cycloalkyl, phenyl or phenyl-loweralkyl group wherein phenyl may be substituted by a halogen atom or a loweralkyl or loweralkoxy group,

X represents an oxygen or sulphur atom,

B represents

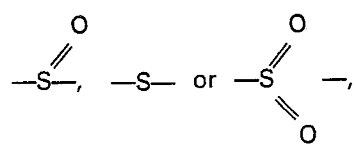

$R^3$ and $R^4$ each represent a hydrogen atom or a loweralkyl, phenyl or phenyl-loweralkyl group wherein phenyl may be substituted by a halogen atom or a loweralkyl or loweralkoxy group and may be the same or different, or $R^3$ and $R^4$ taken together with the adjacent nitrogen may form a heterocyclic residue,

$alk^1$ and $alk^2$ each represent a loweralkylene or loweralkylene-loweralkyl group and may be the same or different, or

a pharmaceutically acceptable addition salt hydrate thereof, the process comprising either

(A) reacting a compound of Formula III

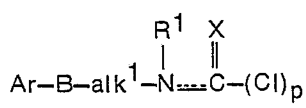

(III)

wherein Ar, B, $alk^1$, $R^1$ and X are defined as above and either p is zero and the dotted line indicates the presence of a double bond or p is one and the dotted line has no significance,

with a compound of Formula IIa

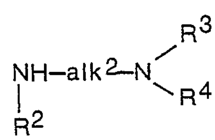

(IIa)

wherein $R^2$, $alk^2$, $R^3$ and $R^4$ are as defined above, with the proviso that when $R^2$ does not represent a hydrogen atom, $R^3$ and $R^4$ must be other than hydrogen or $R^2$ is the same as $R^3$, and $R^4$ is hydrogen; or

(B) when $R^2$ represents a hydrogen atom, reacting a compound of Formula IVa

$$Ar—B—alk^1—NHR^1 \qquad (IVa)$$

wherein Ar, B, $alk^1$ and $R^1$ are as defined above, with a compound of Formula IIb

$$H_2N—alk^2NR^3R^4 \qquad (IIb)$$

wherein $alk^2$, $R^3$ and $R^4$ are as defined above, and with 1,1′-carbonyldiimidazole or 1,1′-thiocarbonyldiimidazole; or

(C) when $R^1$ represents a hydrogen atom, reacting a compound of formula IVb

$$Ar—B—alk^1—NH_2 \qquad (IVb)$$

wherein Ar, B and $alk^1$ are as defined above with a compound of Formula IIb'

$$R^2NH—alk^2—NR^3R^4 \qquad (IIb)$$

wherein $R^2$, $alk^2$, $R^3$ and $R^4$ are as defined above, and with 1,1'-carbonyldiimidazole or 1,1'-thiocarbonyldiimidazole; or

(D) when $R^2$, $R^3$ and $R^4$ are not hydrogen, reacting a compound of Formula V

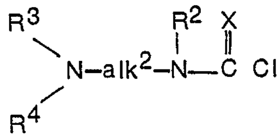

(V)

wherein $R^3$, $R^4$, $alk^2$, $R^2$ and X are as defined above, with a compound of Formula IVa

$$Ar—B—alk^1—NHR^1 \qquad (IVa)$$

wherein Ar, B, $alk^1$ and $R^1$ are as defined above, and with a trisubstituted amine; and if necessary forming a salt or hydrate of such a compound.

2. A process as claimed in Claim 1 for preparing N-[2-[(4-chlorophenyl)thio]ethyl] - N' - [2-(dimethylamino)ethyl] - N - (1-methylethyl)urea, or N-[2-[(4-chlorophenyl)thio]ethyl] - N' - [2-(dimethylamino)-ethyl] - N - (1-methylethyl) urea fumarate [1:1.5].

3. A process as claimed in Claim 1 for preparing N'-[2-(dimethylamino)ethyl] - N - (1-methylethyl) - N - [2-[(2-naphthylene)thio]ethyl]urea, or N'-[2-(dimethylamino)ethyl] - N - (1-methylethyl) - N - [2-[2-naphthylene)thio]ethyl]urea succinate (1:1:5], or N'-[2-(dimethylamino)ethyl] - N - (1-methylethyl) - N - [2-(1-naphthalenylthio)ethyl]urea, or N'-[2-(dimethylamino) ethyl] - N - (1-methylethyl) - N - [2-(1-naphthalenylthio)ethyl]urea, oxalate.

4. A process as claimed in Claim 1 for preparing N-[2-[(4-chlorophenyl)sulphonyl)ethyl] - N' - [2-(dimethylamino)ethyl] - N - (1-methylethyl)urea, or N-[2-[(4-chlorophenyl)sulphonyl)ethyl] - N' - [2-(dimethylamino)ethyl] - N - (1-methylethyl)urea maleate [1:1], or N-[2-[(4-chlorophenyl)sulphonyl ethyl]-N'-[2-(diethylamino)ethyl] - N - (1-methylethyl)urea, or N-[2-[(4-chlorophenyl)sulphonyl]ethyl] - N' - [2-diethylamino)ethyl] - N - (1-methylethyl)urea, tartrate [1:1], or N-[2-[(4-chlorophenyl)sulphonyl]ethyl] - N' - [2-(dimethylamino)ethyl] - N' - (1-methylethyl)urea, or N-[2-[(4-chlorophenyl)sulphonyl]ethyl] - N' - [2-(dimethylamino)ethyl] - N' - (1-methylethyl)urea, maleate [1:1], or N-[2-[(4-chlorophenyl)sulphonyl]-ethyl] - N' - [(dimethylamino)ethyl] - N - (1-methylethyl)thiourea, or N-[2-[(4-chlorophenyl)sulphonyl]-ethyl] - N' - [2-(dimethylamino)ethyl] - N - (1-methylethyl) thiourea, hemifumarate.

5. A process as claimed in Claim 1 for preparing N'[2-(dimethylamino)ethyl] - N - (1-methylethyl) - N - [2-(phenylsulphonyl)ethyl]urea, or N'-[2-(dimethylamino)ethyl] - N - (1-methylethyl) - N - [2-(phenylsulphonyl)ethyl]urea, maleate [1:1], or N'-[2-(diethylamino)ethyl] - N - (1-methylethyl) - N - [2-phenylsulphonyl)ethyl]urea, or N'-[2-(diethylamino)ethyl] - N - (1-methylethyl) - N - [2-(phenylsulphonyl)ethyl]urea, maleate [1:1], or N'-[3-(diethylamino)propyl] - N - (1-methylethyl) - N - [3-(phenylsulphonyl)propyl]urea, or N'-[3-(diethylamino)propyl] - N - (1-methylethyl) - N - [3-(phenylsulphonyl)-propyl]urea, oxalate [1:1] hemihydrate, or N'-[2-(diethylamino)ethyl] - N - (1-methylethyl) - N - [3-(phenylsulphonyl)propyl]urea, or N'-[2-(diethylamino)ethyl] - N - (1-methylethyl) - N - [3-(phenylsulphonyl)propyl]urea oxalate [1:1] hemihydrate, or N'-[3-(diethylamino)propyl] - N - (1-methylethyl) - N - [2-(phenylsulphonyl)ethyl]urea, or N'-[3-(diethylamino)propyl] - N - (1-methylethyl) - N - [2-(phenylsulphonyl)ethyl]urea. 1/4 hydrate, or N'-[2-(diethylamino)ethyl] - N - [2-[(4-fluorophenyl)-sulphonyl]ethyl] - N - (1-methylethyl)urea.

6. A process as claimed in Claim 1 for preparing N'-[2-(dimethylamino)ethyl] - N - (1-methylethyl) - N - [2-[(1-naphthalenesulphonyl)]ethyl]urea, or N'-[2-(dimethylamino)ethyl] - N - (1-methylethyl) - N - [2-[(1-naphthalenesulphonyl)ethyl]urea, maleate.

7. A process as claimed in Claim 1 for preparing N'-[2-(diethylamino)ethyl] - N - (1-methylethyl) - N - [2-[(4-methylphenyl)sulphonyl]ethyl]urea, or N'-[2-(diethylamino)ethyl] - N - (1-methylethyl) - N - [2-[(4-methylphenyl)sulphonyl]ethyl] urea, tartrate, or N-[2-[(3,4-dichlorophenyl)sulphonyl]ethyl] - N' - (sulphonyl]ethyl] - N' - (dimethylamino)ethyl] - N - (1-methylethyl)urea, or N-[2-[(3,4-dichlorophenyl)-sulphonyl]ethyl] - N' - (dimethylamino)ethyl] - N - (1-methylethyl)urea maleate.

8. A process as claimed in Claim 1 for preparing N-[2-[(4-chlorophenyl)sulphinyl]ethyl] - N' - [2-(dimethylamino)ethyl] - N - (1-methylethyl)urea, or N-[2-[(4-chlorophenyl)sulphinyl]ethyl] - N' - [2-(dimethylamino)ethyl] - N - (1-methylethyl)urea fumarate [1:1], or N'-[2-(diethylamino)ethyl] - N - (1-methylethyl) - N - [2-[(4-methylphenyl)sulphinyl]ethyl]urea, or N'-[2-(diethylamino)ethyl] - N - (1-methylethyl) - N - [2-[(4-methylphenyl)sulfinyl]ethyl]urea hemihydrate.

9. A process for preparing a therapeutic composition for the treatment of cardiac arrhythmias comprising admixing (a) an effective amount of a compound having the formula

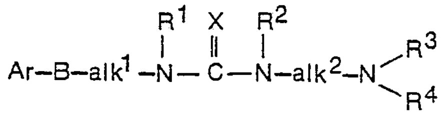

wherein:

Ar represents a 1 or 2-naphthyl, 2,3-dihydro-1H-inden-4(or 5)yl, 2-furanyl, phenyl or substituted phenyl group the said substituted phenyl group being substituted by 1—3 radicals which may be the same or different which radicals are selected from loweralkyl, loweralkoxy, halogen, trifluoromethyl, nitro, cyano, or

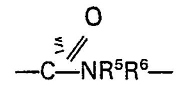

groups, wherein $R^5$ and $R^6$ each represents a hydrogen atom or a loweralkyl group, and Ar may include one intervening methylene group attached to B,

$R^1$ and $R^2$ each represent a hydrogen atom or a loweralkyl, cycloalkyl, phenyl or phenyl-loweralkyl group wherein phenyl may be substituted by a halogen atom or a loweralkyl or loweralkoxy group,

X represents an oxygen or sulphur atom,

B represents

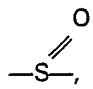 —S— or 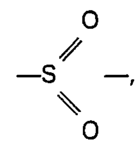

$R^3$ and $R^4$ each represent a hydrogen atom or a loweralkyl, phenyl or phenyl-loweralkyl group wherein phenyl may be substituted by a halogen atom or a loweralkyl or loweralkoxy group and may be the same or different, or $R^3$ and $R^4$ taken together with the adjacent nitrogen may form a heterocyclic residue,

$alk^1$ and $alk^2$ each represent a loweralkylene or loweralkylene-loweralkyl group and may be the same or different, or

a pharmaceutically acceptable addition salt or hydrate thereof, and (b) a pharmaceutically acceptable carrier therefor.

10. A process for preparing a therapeutic composition for the treatment of cardiac arrhythmias comprising admixing (a) an effective amount of a compound named in any one of Claims 2 to 8, or a pharmaceutically acceptable addition salt or hydrate thereof, and (b) a pharmaceutically acceptable carrier therefor.

**Revendications Pour les Etats Contractants: BE CH DE FR GB IT LI LU NL SE**

1. Composé de formule:

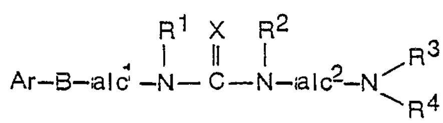

où Ar représente un radical 1- ou 2-naphtyle, 2,3-dihydro-1H-indène-4(ou 5)yle, 2-furanyle, phényle ou phényle substitué, le radical phényle substitué étant substitué par 1—3 radicaux qui peuvent être identiques ou différents et sont choisis parmi les radicaux alcoyle inférieurs, alcoxy inférieurs, halogéno, trifluorométhyle, nitro, cyano et —C(O)—$NR^5R^6$, où $R^5$ et $R^6$ représentent chacun un atome d'hydrogène ou un radical alcoyle inférieur, et Ar peut comprendre un radical méthylène intermédiaire uni à B,

$R^1$ et $R^2$ représentent chacun un atome d'hydrogène ou un radical alcoyle inférieur, cycloalcoyle, phényle ou phényl-alcoyle inférieur, où le radical phényle peut être substitué par un atome d'halogène ou un radical alcoyle inférieur, ou alcoxy inférieur,

X représente un atome d'oxygène ou de soufre,

B représente un radical thio, sulfinyle ou sulfonyle,

$R^3$ et $R^4$ représentent chacun un atome d'hydrogène ou un radical alcoyle inférieur, phényle ou phényl-alcoyle inférieur, où le radical phényle peut être substitué par un atome d'halogène ou un radical alcoyle inférieur ou alcoxy inférieur, et peuvent être identiques ou différents, ou bien $R^3$ et $R^4$ pris ensemble avec l'atome d'azote adjacent peuvent former un reste hétérocyclique,

$alc^1$ et $alc^2$ représentent chacun un radical alcoyléne inférieur ou alcoylène(inférieur)-alcoyle inférieur

et peuvent être identiques ou différents, et les sels d'addition pharmaceutiquement acceptables et hydrates de ce composé.

2. Composé suivant la revendication 1 qui est la N-[2-[(4-chlorophényl)thio]éthyl] - N' - [2-(diméthylamino)éthyl] - N - (1-méthyléthyl)urée, ou le fumarate de N-[2-[(4-chlorophényl)thio]éthyl] - N' - [2-(diméthylamino)éthyl] - N - (1-méthyléthyl) urée [1,5:1].

3. Composé suivant la revendication 1 qui est la N'-[2-(diméthylamino)éthyl] - N - (1-méthyléthyl) - N - [2-[(2-naphthylène)thio]éthyl]urée ou le succinate de N'-[2-(diméthylamino)éthyl] - N - (1-méthyléthyl) - N - [2-[2-naphthylène)thio]éthyl]urée [1,5:1] ou bien la N'-[2-(diméthylamino)éthyl]-N-(1-méthyléthyl)-N-[2-(1-naphtalénylthio)éthyl]-urée ou l'oxalate de N'-[2-(diméthylamino)éthyl] - N - (1-méthyléthyl) - N - [2-(1-naphtalénylthio)éthyl]urée.

4. Composé suivant la revendication 1 qui est la N-[2-[(4-chlorophényl)sulfonyl)éthyl] - N' - [2-(diméthylamino)éthyl] - N - (1-méthyléthyl)urée ou le maléate de N-[2-[(4-chlorophényl)sulfonyl]éthyl] - N' - [2-(diméthylamino)éthyl] - N - (1-méthyléthyl)urée [1:1], ou la N-[2-[(4-chlorophényl)sulfonyl]éthyl]-N'-[2-(diéthylamino)éthyl] - N - (1-méthyléthyl)urée ou le tartrate de N-[2-[(4-chlorophényl)sulfonyl]-éthyl] - N' - [2-diéthylamino)éthyl] - N - (1-méthyléthyl)urée [1:1], ou la N-[2-[(4-chlorophényl)sulfonyl]-éthyl] - N' - [2-(diméthylamino)éthyl] - N' - (1-méthyléthyl)urée ou le maléate de N-[2-[(4-chlorophényl)-sulfonyl]éthyl] - N' - [2-(diméthylamino)éthyl] - N' - (1-méthyléthyl)urée [1:1], ou la N-[2-[(4-chlorophényl)sulfonyl]éthyl] - N' - [2-(diméthylamino)éthyl] - N - (1-méthyléthyl)thiourée, ou l'hemifumarate de N-[2-[(4-chlorophényl)sulfonyl]éthyl] - N' - [2-(diméthylamino)éthyl] - N - (1-méthyléthyl) thiourée.

5. Composé suivant la revendication 1 qui est la N'-[2-(diméthylamino)éthyl] - N - (1-méthyléthyl) - N - [2-(phénylsulfonyl)éthyl]urée ou le maléate de N'-[2-(diméthylamino)éthyl] - N - (1-méthyléthyl) - N - [2-(phénylsulfonyl)éthyl]urée [1:1], ou la N'-[2-(diéthylamino)éthyl] - N - (1-méthyléthyl) - N - [2-(phényl-sulfonyl)éthyl]urée ou le maléate de N'-[2-(diéthylamino)éthyl] - N - (1-méthyléthyl) - N - [2-(phényl-sulfonyl)éthyl]urée [1:1], ou la N'-[3-(diéthylamino)propyl] - N - (1-méthyléthyl) - N - [3-(phénylsulfonyl)-propyl]urée ou l'oxalate de N'-[3-(diéthylamino)propyl] - N - (1-méthyléthyl) - N - [3-(phénylsulfonyl)-propyl]urée [1:1] hémihydraté ou la N'-[2-(diéthylamino)éthyl] - N - (1-méthyléthyl) - N - [3-(phényl-sulfonyl)propyl]urée ou l'oxalate de N'-[2-(diéthylamino)éthyl] - N - (1-méthyléthyl) - N - [3-(phényl-sulfonyl)propyl]urée [1:1] hémihydraté ou la N'-[3-(diéthylamino)propyl] - N - (1-méthyléthyl) - N - [2-(phénylsulfonyl)éthyl]urée ou la N'-[3-(diéthylamino)propyl] - N - (1-méthyléthyl) - N - [2-(phényl-sulfonyl)éthyl]urée tétarto-hydratée ou la N'-[2-(diéthylamino)éthyl] - N - [2-[(4-fluorophényl)sulfonyl]-éthyl] - N - (1-méthyléthyl)urée.

6. Composé suivant la revendication 1 qui est la N'-[2-(diméthylamino)éthyl] - N - (1-méthyléthyl) - N - [2-[(1-naphtalènesulfonyl)]éthyl]urée ou le maléate de N'-[2-(diméthylamino)éthyl] - N - (1-méthyléthyl) - N - [2-[(1-naphtalènesulfonyl)]éthyl]urée.

7. Composé suivant la revendication 1 qui est la N'-[2-(diéthylamino)éthyl] - N - (1-méthyléthyl) - N - [2-[(4-méthylphényl)sulfonyl]éthyl]urée ou le tartrate de N'-[2-(diéthylamino)éthyl] - N - (1-méthyléthyl) - N - [2-[(4-méthylphényl)sulfonyl]éthyl]urée ou la N-[2-[(3,4-dichlorophényl)sulfonyl]éthyl] - N' - (diméthylamino)éthyl] - N - (1-méthyléthyl)urée ou le maléate de N-[2-[(3,4-dichlorophényl)sulfonyl]-éthyl] - N' - (diméthylamino)éthyl] - N - (1-méthyléthyl)urée.

8. Composé suivant la revendication 1 qui est la N-[2-[(4-chlorophényl)sulfinyl]éthyl] - N' - [2-(diméthylamino)éthyl] - N - (1-méthyléthyl)urée ou le fumarate de N-[2-[(4-chlorophényl)sulfinyl]éthyl] - N' - [2-(diméthylamino)éthyl] - N - (1-méthyléthyl)urée [1:1] ou la N'-[2-(diéthylamino)éthyl] - N - (1-méthyléthyl) - N - [2-[(4-méthylphényl)sulfinyl]éthyl]urée ou la N'-[2-(diéthylamino)éthyl] - N - (1-méthyléthyl) - N - [2-[(4-méthylphényl)sulfinyl]éthyl]urée hémihydratée.

9. Composé suivant l'une quelconque des revendications 1 à 8 à utiliser pour le traitement des arythmies cardiaques.

10. Composition thérapeutique pour le traitement des arythmies cardiaques, comprenant (a) une quantité efficace d'une composé suivant l'une quelconque des revendications 1 à 9 et (b) un excipient pharmaceutiquement acceptable.

**Revendications pour l'Etat Contractant: AT**

1. Procédé de préparation d'un composé de formule:

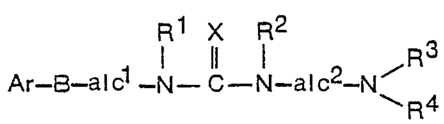

où Ar représente un radical 1- ou 2-naphtyle, 2,3-dihydro-1H-indène-4(ou 5)yle, 2-furanyle, phényle ou phényle substitué, le radical phényle substitué étant substitué par 1—3 radicaux qui peuvent être identiques ou différents et sont choisis parmi les radicaux alcoyle inférieurs, alcoxy inférieurs, halogéno, trifluorométhyle, nitro, cyano et —C(O)—$NR^5R^6$, où $R^5$ et $R^6$ représentent chacun un atome d'hydrogène ou un radical alcoyle inférieur, et Ar peut comprendre un radical méthylène intermédiaire uni à B,

$R^1$ et $R^2$ représentent chacun un atome d'hydrogène ou un radical alcoyle inférieur, cycloalcoyle, phényle ou phényl-alcoyle inférieur, où le radical phényle peut être substitué par un atome d'halogène ou un radical alcoyle inférieur ou alcoxy inférieur,

X représente un atome d'oxygène ou de soufre,

B représente un radical thio, sulfinyle ou sulfonyle,

$R^3$ et $R^4$ représentent chacun un atome d'hydrogène ou un radical alcoyle inférieur, phényl ou phényl-alcoyle inférieur, où le radical phényle peut être substitué par un atome d'halogène ou un radical alcoyle inférieur ou alcoxy inférieur, et peuvent être identiques ou différents, ou bien $R^3$ et $R^4$ pris ensemble avec l'atome d'azote adjacent peuvent former un reste hétérocyclique,

$alc^1$ et $alc^2$ représentent chacun un radical alcoyléne inférieur ou alcoylène(inférieur)-alcoyle inférieur et peuvent être identiques ou différents, et les sels d'addition pharmaceutiquement acceptables et hydrates de ce composé,

Le procédé comprenant

(A) la réaction d'un composé de formule III

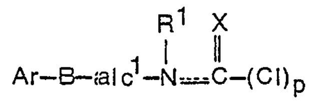

(III)

où Ar, B, $alc^1$, $R^1$ et X sont tels que définis ci-dessus et soit p représente zéro et la ligne en pointillé représente la présence d'une double liaison, soit p représente un et la ligne en pointillé n'a pas de signification,

avec un composé de formule IIa

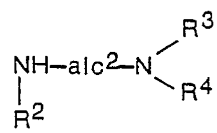

(IIa)

où $R^2$, $alc^2$, $R^3$ et $R^4$ sont tels que définis ci-dessus, avec la restriction que lorsque $R^2$ ne représente pas un atome d'hydrogène, $R^3$ et $R^4$ doivent être autres que l'hydrogène ou $R^2$ est le même que $R^3$, et $R^4$ représente un atome d'hydrogène; ou

(B) lorsque $R^2$ représente un atome d'hydrogène, la réaction d'un composé de formule IVa

$$Ar—B—alc^1—NHR^1$$

où Ar, B, $alc^1$ et $R^1$ sont tels que définis ci-dessus, avec un composé de formule IIb

$$H_2N—alc^2NR^3R^4$$

où $alc^2$, $R^3$ et $R^4$ sont tels que définis ci-dessus, et avec le 1,1'-carbonyldiimidazole ou le 1,1'-thiocarbonyldiimidazole; ou

(C) lorsque $R^1$ représente un atome d'hydrogène, la réaction d'un composé de formule IVb

$$Ar—B—alc^1—NH_2 \qquad (IVb)$$

où Ar, B et $alc^1$ sont tels que définis ci-dessus, avec un composé de formule IIb'

$$R^2NH—alc^2—NR^3R^4 \qquad (IIb')$$

où $R^2$, $alc^2$, $R^3$ et $R^4$ sont tels que définis ci-dessus, et avec le 1,1'-carbonyldiimidazole ou le 1,1'-thiocarbonyldiimidazole; ou

(D) lorsque $R^2$, $R^3$ et $R^4$ ne représentent pas des atomes d'hydrogène, la réaction d'un composé de formule V

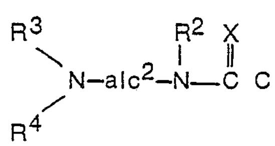

(V)

où $R^3$, $R^4$, $alc^2$, $R^2$ et X sont tels que définis ci-dessus, avec un composé de formule IVa

$$Ar—B—alc^1—NHR^1 \qquad (IVa)$$

où Ar, B, $alc^1$ et $R^1$ sont tels que définis ci-dessus, et avec une amine trisubstituée; et, si nécessaire, la formation d'un sel ou hydrate d'un tel composé.

2. Procédé suivant la revendication 1 de préparation de la N-[2-[(4-chlorophényl)thio]éthyl] - N' - [2-(diméthylamino)éthyl] - N - (1-méthyléthyl)urée, ou du fumarate de N-[2-[4-chlorophényl)thio]éthyl] - N' - [2-(diméthylamino)éthyl] - N - (1-méthyléthyl) urée [1,5:1].

3. Procédé suivant la revendication 1 de préparation de la N'-[2-(diméthylamino)éthyl] - N - (1-méthyléthyl) - N - [2-[(2-naphthylène)thio]éthyl]urée ou du succinate de N'-[2-(diméthylamino)éthyl] - N - (1-méthyléthyl) - N - [2-[(2-naphtylène) thio]éthyl]urée [1,5:1] ou bien de la N'-[2-(diméthylamino)éthyl]-N-(1-méthyléthyl)-N-[2-(1-naphtalénylthio]éthyl]urée ou de l'oxalate de N'-[2-(diméthylamino)éthyl] - N - (1-méthyléthyl) - N - [2-(1-naphthalenylthio)éthyl]urée.

4. Procédé suivant la revendication 1 de préparation de la N-[2-[(4-chlorophényl)sulfonyl]éthyl] - N' - [2-(diméthylamino)éthyl] - N - (1-méthyléthyl)urée ou du maléate de N-[2-[(4-chlorophényl)sulfonyl]-éthyl] - N' - [2-(diméthylamino)éthyl] - N - (1-méthyléthyl)urée [1:1], ou de la N-[2-[(4-chlorophényl)-sulfonyl]- éthyl]-N'-[2-(diéthylamino)éthyl] - N - (1-méthyléthyl)urée ou du tartrate de N-[2-[(4-chlorophényl)sulfonyl]éthyl] - N' - [2-diéthylamino)éthyl] - N - (1-méthyléthyl)urée [1:1], ou de la N-[2-[(4-chlorophényl)sulfonyl]éthyl] - N' - [2-(diméthylamino)éthyl] - N' - (1-méthyléthyl)urée ou du maléate de N-[2-[(4-chlorophényl)sulfonyl]éthyl] - N' - [2-(diméthylamino)éthyl] - N' - (1-méthyléthyl)urée [1:1] ou de la N-[2-[(4-chlorophényl)sulfonyl]éthyl] - N' - [2-(diméthylamino)éthyl] - N - (1-méthyléthyl)-thiourée, ou de l'hémifumarate de N-[2-[(4-chlorophényl)sulfonyl)éthyl] - N' - [2-(diméthylamino)éthyl] - N - (1-méthylethyl) thiourée.

5. Procédé suivant la revendication 1 de préparation de la N'-[2-(diméthylamino)éthyl] - N - (1-méthyléthyl) - N - [2-(phénylsulfonyl)éthyl]urée ou du maléate de N'-[2-(diméthylamino)éthyl] - N - (1-méthyléthyl) - N - [2-(phénylsulfonyl)éthyl]urée [1:1], ou de la N'-[2-(diéthylamino)éthyl] - N - (1-méthyléthyl) - N - [2-phénylsulfonyl)éthyl]urée ou du maléate de N'-[2-(diéthylamino)éthyl] - N - (1-méthyléthyl) - N - [2-(phénylsulfonyl)éthyl]urée [1:1], ou de la N'-[3-(diéthylamino)propyl] - N - (1-méthyléthyl) - N - [3-(phénylsulfonyl)propyl]urée ou de l'oxalate de N'-[3-(diéthylamino)propyl] - N - (1-méthyléthyl) - N - [3-(phénylsulfonyl)propyl]urée [1:1] hémihydraté ou de la N'-[2-(diéthylamino)éthyl] - N - (1-méthyléthyl) - N - [3-(phénylsulfonyl)propyl]urée ou de l'oxalate de N'-[2-(diéthylamino)éthyl] - N - (1-méthyléthyl) - N - [3-(phénylsulfonyl)propyl]urée [1:1] hémihydraté ou de la N'-[3-(diéthylamino)-propyl] - N - (1-méthyléthyl) - N - [2-(phénylsulfonyl)éthyl]urée ou de la N'-[3-(diéthylamino)propyl] - N - (1-méthyléthyl) - N - [2-(phénylsulfonyl)éthyl]urée tétarto-hydratée ou de la N'-[2-(diéthylamino)éthyl] - N - [2-[(4-fluorophényl)sulfonyl]éthyl] - N - (1-méthyléthyl)urée.

6. Procédé suivant la revendication 1 de préparation de la N'-[2-(diméthylamino)éthyl] - N - (1-méthyléthyl) - N - [2-[(1-naphtalènesulfonyl)éthyl]urée ou du maléate de N'-[2-(diméthylamino)éthyl] - N - (1-méthyléthyl) - N - [2-[(1-naphtalènesulfonyl)]éthyl]urée.

7. Procédé suivant la revendication de préparation de la N'-[2-(diéthylamino)éthyl] - N - (1-méthyléthyl) - N - [2-[(4-méthylphényl)sulfonyl]éthyl]urée ou du tartrate de N'-[2-(diéthylamino)éthyl] - N - (1-méthyléthyl) - N - [2-[(4-méthylphényl)sulfonyl]éthyl] urée ou de la N-[2-[(3,4-dichlorophényl)sulfonyl]-éthyl] - N' - [(diméthylamino)éthyl] - N - (1-méthyléthyl)urée ou du maléate de N-[2-[(3,4-dichlorophényl)sulfonyl]éthyl] - N' - [(diméthylamino)éthyl] - N - (1-méthyléthyl)urée.

8. Procédé suivant la revendication 1 de préparation de la N-[2-[(4-chlorophényl)sulfinyl]éthyl] - N' - [2-(diméthylamino)éthyl] - N - (1-méthylethyl)urée ou du fumarate de N-[2-[(4-chlorophényl)sulfinyl]-éthyl] - N' - [2-(diméthylamino)éthyl] - N - (1-méthyléthyl)urée [1:1] ou de la N'-[2-(diéthylamino)éthyl] - N - (1-méthyléthyl) - N - [2-[(4-méthylphényl)sulfinyl]éthyl]urée ou du la N'-[2-(diéthylamino)éthyl] - N - (1-méthyléthyl) - N - [2-[(4-méthylphényl)sulfinyl]éthyl]urée hémihydratée.

9. Procédé de préparation d'une composition thérapeutique pour le traitement des arythmics cardiaques, qui comprend le mélange (a) d'une quantité efficace d'un composé de formule

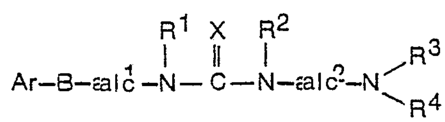

où Ar représente un radical 1- ou 2-naphtyle, 2,3-dihydro-1H-indène-4(ou 5)yle, 2-furanyle, phényle ou phényle substitué, le radical phényle substitué étant substitué par 1—3 radicaux qui peuvent être identiques ou différents et sont choisis parmi les radicaux alcoyle inférieurs, alcoxy inférieurs, halogéno, trifluorométhyle, nitro, cyano et —C(O)—$NR^5R^6$, où $R^5$ et $R^6$ représentent chacun un atome d'hydrogène ou un radical alcoyle inférieur, et Ar peut comprendre un radical méthylène intermédiaire uni à B,

$R^1$ et $R^2$ représentent chacun un atome d'hydrogène ou un radical alcoyle inférieur, cycloalcoyle, phényle ou phényl-alcoyle inférieur, où le radical phényle peut être substitué par un atome d'halogène ou un radical alcoyle inférieur ou alcoxy inférieur,

X représente un atome d'oxygène ou de soufre,

B représente un radical thio, sulfinyle ou sulfonyle,

$R^3$ et $R^4$ représentent chacun un atome d'hydrogène ou un radical alcoyle inférieur, phényl ou phényl-alcoyle inférieur, où le radical phényl peut être substitué par un atome d'halogène ou un radical alcoyle inférieur ou alcoxy inférieur, et peuvent être identiques ou différents, ou bien $R^3$ et $R^4$ pris ensemble avec l'atome d'azote adjacent peuvent former un reste hétérocyclique,

alc[1] et alc[2] représentent chacun un radical alcoyléne inférieur ou alcoylène(inférieur)-alcoyle inférieur et peuvent être identiques ou différents, ou d'un sel d'addition pharmaceutiquement acceptable ou hydrate de ce composé, et (b) d'un excipient pharmaceutiquement acceptable.

10. Procédé de préparation d'une composition thérapeutique pour le traitement des arythmics cardiaques, qui comprend le mélange (a) d'une quantité efficace d'un composé cité dans l'une quelconque des revendications 1 à 8, ou d'un sel d'addition pharmaceutiquement acceptable ou hydrate de ce composé, et (b) d'un excipient pharmaceutiquement acceptable.

**Patentansprüche Für die Vertragsstaaten: BE CH DE FR GB IT LI LU NL SE**

Verbindung der Formel

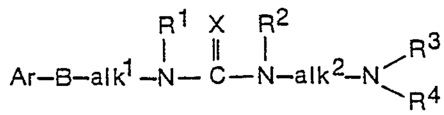

worin

Ar eine 1- oder 2-Naphthyl-, 2,3-Dihydro-1H-inden-4(oder 5-yl-, 2-Furanyl-, Phenyl- oder substituierte Phenylgruppe bedeutet, wobei die substituierte Phenylgruppe durch 1 bis 3 Radikale substituiert ist, welche gleich oder verschieden sein können und aus Niederalkyl-, Niederalkoxygruppen, Halogen, Trifluormethyl-, Nitro-, Cyan- oder

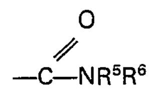

-Gruppen, worin $R^5$ und $R^6$ je ein Wasserstoffatom oder eine Niederalkylgruppe darstellen, ausgewählt sind, und Ar eine an B gebundene dazwischenliegende Methylengruppe einschließen kann;

$R^1$ und $R^2$ je ein Wasserstoffatom oder eine Niederalkyl-, Cycloalkyl-, Phenyl- oder Phenylniederalkylgruppe bedeuten, worin Phenyl durch ein Halogenatom oder eine Niederalkyl- oder Niederalkoxygruppe substituiert sein kann;

X ein Sauerstoff- oder Schwefelatom bedeutet;

B für

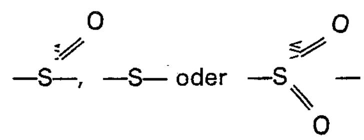
, oder

— steht;

$R^3$ und $R^4$ je ein Wasserstoffatom oder eine Niederalkyl-, Phenyl- oder Phenylniederalkylgruppe bedeuten, worin Phenyl durch ein Halogenatom oder eine Niederalkyl-oder Niederalkoxygruppe substituiert sein kann, und gleich oder verschieden sein können oder $R^3$ und $R^4$ zusammen mit dem anliegenden Stickstoff einen heterocyclischen Rest bilden können;

alk[1] und alk[2] je eine Niederalkylen- oder Niederalkylenniederalkylgruppe bedeuten und gleich oder verschieden sein können;

sowie deren pharmazeutisch annehmbaren Additions-salze und Hydrate.

2. Verbindung nach Anspruch 1, die N-[2-[(4-Chlorphenyl)thio]ethyl] - N' - [2-(dimethylamino)ethyl] - N - (1-methylethyl)harnstoff oder N-[2-[(4-Chlorphenyl)thio]ethyl] - N' - [2-(dimethylamino)ethyl] - N - (1-methylethyl) harnstoffumarat [1:1.5].

3. Verbindung nach Anspruch 1, die N'-[2-(Dimethylamino)ethyl] - N - (1-methylethyl) - N - [2-[(2-naphthyl)-thio]ethyl]harnstoff oder N'-[2-(Dimethylamino)ethyl] - N - (1-methylethyl) - N - [2-[2-naphthyl)thio]ethyl]harnstoffsuccinat [1:1.5] oder N'-[2-(Dimethylamino)ethyl] - N - (1-methylethyl) - N - [2-(1-naphthylthio)ethyl]harnstoff oder N'-[2-(Dimethylamino) ethyl] - N - (1-methylethyl) - N - [2-(1-naphthylthio)ethyl]harnstoffoxalat ist.

4. Verbindung nach Anspruch 1, die N-[2-[(4-Chlorphenyl)sulfonyl)ethyl] - N' - [2-(dimethylamino)-ethyl] - N - (1-methylethyl)harnstoff oder N-[2-[(4-Chlorphenyl)sulfonyl)ethyl] - N' - [2-(dimethylamino)ethyl] - N - (1-methylethyl)harnstoffmaleat [1:1] oder N-[2-[(4-Chlorphenyl)sulfonyl)ethyl]-N'-[2-(diethylamino)ethyl] - N - (1-methylethyl)harnstoff oder N-[2-[(4-Chlorphenyl)sulfonyl]ethyl] - N' - [2-(diethylamino)ethyl] - N - (1-methylethyl)harnstofftartrat [1:1] oder N-[2-[(4-Chlorphenyl)sulfonyl]ethyl] - N' - [2-(dimethylamino)ethyl] - N' - (1-methylethyl)harnstoff oder N-[2-[(4-Chlorphenyl)sulfonyl]ethyl] - N' - [2-(dimethylamino)ethyl] - N' - (1-methylethyl)harnstoffmaleat [1:1] oder N-[2-[(4-Chlorphenyl)-sulfonyl]ethyl] - N' - [(dimethylamino)ethyl] - N - (1-methylethyl)thioharnstoff oder N-[2-[(4-Chlorphenyl)-sulfonyl]ethyl] - N' - [2-(dimethylamino)ethyl] - N - (1-methylethyl)-thioharnstoffhemifumarat ist.

5. Verbindung nach Anspruch 1, die N'-[2-(Dimethylamino)ethyl] - N - (1-methylethyl) - N - [2-(phenylsulfonyl)-ethyl]harnstoff oder N'-[2-(Dimethylamino)ethyl] - N - (1-methylethyl) - N - [2-(phenylsulfonyl)ethyl]harnstoffmaleat [1:1] oder N'-[2-(Diethylamino)ethyl] - N - (1-methylethyl) - N - [2-phenylsulfonyl)ethyl]harnstoff oder N'-[2-(Diethylamino)ethyl] - N - (1-methylethyl) - N - [2-(phenylsulfonyl)ethyl]harnstoffmaleat [1:1] oder N'-[3-(diethylamino)propyl] - N - (1-methylethyl) - N - [3-(phenylsulfonyl)propyl]harnstoff oder N'-[3-(Diethylamino)propyl] - N - (1-methylethyl) - N - [3-(phenylsulfonyl)propyl]harnstoffoxalat-[1:1]-hemihydrat oder N'-[2-(Diethylamino)ethyl] - N - (1-methylethyl) - N - [3-(phenylsulfonyl)propyl]harnstoff oder N'-[2-(Diethylamino)ethyl] - N - (1-methylethyl) - N - [3-(phenylsulfonyl)propyl]harnstoffoxalat-[1:1]-hemihydrat oder N'-[3-(Diethylamino)propyl] - N - (1-methylethyl) - N - [2-(phenylsulfonyl)ethyl]harnstoff oder N'-[3-(Diethylamino)propyl] - N - (1-methylethyl) - N - [2-(phenylsulfonyl)ethyl]harnstoff. 1/4 Hydrat oder N'-[2-(Diethylamino)ethyl] - N - [2-[(4-fluorphenyl)-sulfonyl]ethyl] - N - (1-methylethyl)harnstoff ist.

6. Verbindung nach Anspruch 1, die N'-[2-(Dimethylamino)ethyl] - N - (1-methylethyl) - N - [2-[(1-naphthylsulfonyl)]ethyl]harnstoff oder N'-[2-(Dimethylamino)ethyl] - N - (1-methylethyl) - N - [2-[(1-naphthyl)sulfonyl]ethyl]-harnstoffmaleat ist.

7. Verbindung nach Anspruch 1, die N'-[2-(Diethylamino)ethyl] - N - (1-methylethyl) - N - [2-[(4-methylphenyl)sulfonyl]ethyl]harnstoff oder N'-[2-(Diethylamino)ethyl] - N - (1-methylethyl) - N - [2-[(4-methylphenyl)sulfonyl]ethyl]-harnstofftartrat oder N-[2-[(3,4-Dichlorphenyl)sulfonyl]ethyl] - N' - (dimethylamino)ethyl] - N - (1-methylethyl)harnstoff oder N-[2-[(3,4-Dichlorphenyl)sulfonyl]ethyl] - N' - (dimethylamino)ethyl] - N - (1-methylethyl)harnstoffmaleat ist.

8. Verbindung nach Anspruch 1, die N-[2-[(4-Chlorphenyl)sulfinyl]ethyl] - N' - [2-(dimethylamino)ethyl] - N - (1-methylethyl)harnstoff oder N-[2-[(4-Chlorphenyl)sulfinyl]ethyl] - N' - [2-(dimethylamino)ethyl] - N - (1-methylethyl)harnstoffumarat [1:1] oder N'-[2-(Diethylamino)ethyl] - N - (1-methylethyl) - N - [2-[(4-methylphenyl)sulfinyl]ethyl]harnstoff oder N'-[2-(Diethylamino)ethyl] - N - (1 - methylethyl) - N - [2-[(4-methylphenyl)sulfinyl]ethyl]harnstoffhemihydrat ist.

9. Verbindung nach einem der Ansprüche 1 bis 8 zur Verwendung bei der Behandlung von Herzrhythmusstörungen.

10. Therapeutische Zusammensetzung für die Behandlung von Herzrhythmusstörungen, enthaltend
(a) eine wirksame Menge einer Verbindung nach einem der Ansprüche 1 bis 9 und
(b) einen pharmazeutisch annehmbaren Träger dafür.

**Patentansprüche für den Vertragsstaat: AT**

1. Verfahren zur Herstellung einer Verbindung der Formel

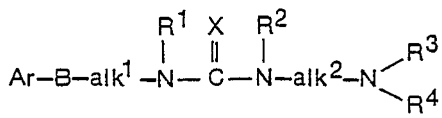

worin

Ar eine 1- oder 2-Naphthyl-, 2,3-Dihydro-1H-inden-4(oder 5)-yl-, 2-Furanyl-, Phenyl- oder substituierte Phenylgruppe bedeutet, wobei die substituierte Phenylgruppe durch 1 bis 3 Radikale substituiert ist, welche gleich oder verschieden sein können und aus Niederalkyl-, Niederalkoxygruppen, Halogen, Trifluormethyl-, Nitro-, Cyan- oder

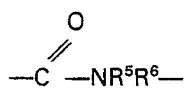

Gruppen, worin $R^5$ und $R^6$ je ein Wasserstoffatom oder eine Niederalkylgruppe darstellen, ausgewählt sind, und Ar eine an B gebundene dazwischenliegende Methylengruppe einschließen kann;

$R^1$ und $R^2$ je ein Wasserstoffatom oder eine Niederalkyl-, Cycloalkyl-, Phenyl- oder Phenylniederalkylgruppe bedeuten, worin Phenyl durch ein Halogenatom oder eine Niederalkyl- oder Niederalkoxygruppe substituiert sein kann;

X ein Sauerstoff- oder Schwefelatom bedeutet;

B für

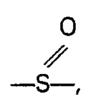, —S— oder 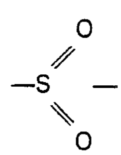

steht;

$R^3$ und $R^4$ je ein Wasserstoffatom oder eine Niederalkyl-, Phenyl- oder Phenylniederalkylgruppe bedeuten, worin Phenyl durch ein Halogenatom oder eine Niederalkyl-oder Niederalkoxygruppe substituiert sein kann, und gleich oder verschieden sein können oder $R^3$ und $R^4$ zusammen mit dem anliegenden Stickstoff einen heterocyclischen Rest bilden können;

$alk^1$ und $alk^2$ je eine Niederalkylen- oder Niederalkylenniederalkylgruppe bedeuten und gleich oder verschieden sein können;

oder von deren pharmazeutisch annehmbaren Additionssalz oder Hydrat, dadurch gekennzeichnet, daß man

(A) eine Verbindung der Formel

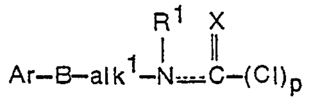

(III)

worin Ar, B, $alk^1$, $R^1$ und X wie vorstehend definiert sind und entweder p gleich Null ist und die strichlierte Linie die Gegenwart einer Doppelbindung anzeigt oder p gleich 1 ist und die strichlierte Linie ohne Bedeutung ist, mit einer Verbindung der Formel

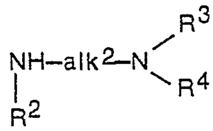

(IIa)

worin $R^2$, $alk^2$, $R^3$ und $R^4$ wie vorstehend definiert sind, umsetzt, mit der Maßgabe, daß dann, wenn $R^2$ von Wasserstoff verschieden ist, $R^3$ und $R^4$ von Wasserstoff verschieden sein müssen oder dann, wenn $R^2$ gleich ist wie $R^3$, $R^4$ Wasserstoff ist; oder

(B) wenn $R^2$ ein Wasserstoffatom bedeutet, eine Verbindung der Formel

$$Ar—B—alk^1—NHR^1, \qquad (IVa)$$

worin Ar, B, $alk^1$ und $R^1$ wie vorstehend definiert sind, mit einer Verbindung der Formal

$$H_2N—alk^2NR^3R^4, \qquad (IIb)$$

worin $alk^2$, $R^3$ und $R^4$ wie vorstehend definiert sind, und mit 1,1'-Carbonyldiimidazol oder 1,1'-Thiocarbonyldiimidazol umsetzt; oder

(C) wenn $R^1$ ein Wasserstoffatom bedeutet, eine Verbindung der Formel

$$Ar—B—alk^1—NH_2, \qquad (IVb)$$

worin Ar, B und $alk^1$ wie vorstehend definiert sind, mit einer Verbindung der Formel

$$R^2NH—alk^2—NR^3R^4, \qquad (IIb')$$

worin $R^2$, $alk^2$, $R^3$ und $R^4$ wie vorstehend definiert sind, und mit 1,1'-Carbonyldiimidazol oder 1,1'-Thiocarbonyldiimidazol umsetzt; oder

(D) wenn $R^2$, $R^3$ und $R^4$ von Wasserstoff verschieden sind, eine Verbindung der Formel

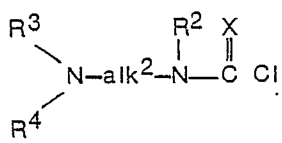

(V)

worin $R^3$, $R^4$, $alk^2$, $R^2$ und X wie vorstehend definiert sind, mit einer Verbindung der Formel

$$Ar—B—alk^1—NHR^1, \qquad (IVa)$$

worin Ar, B, $alk^1$ und $R^1$ wie vorstehend definiert sind;
und mit einem trisubstituierten Amin umsetzt;

wonach man nötigenfalls eine so erhaltene Verbindung in ein Salz oder Hydrat überführt.

2. Verfahren nach Anspruch 1, zur Herstellung von N - [2 - [(4 - Chlorphenyl)thio]ethyl] - N' - [2 - [2 - (dimethylamino)ethyl] - N - (1 - methylethyl)harnstoff oder N - [2 - [(4 - Chlorphenyl)thio]ethyl] - N' - [2 - (dimethylamino)ethyl] - N - (1 - methylethyl)harnstoffumarat [1:1.5].

3. Verfahren nach Anspruch 1, zur Herstellung von N' - [2 - (Dimethylamino)ethyl] - N - (1 - methylethyl) - N - [2 - [(2 - naphthyl) - thio]ethyl]harnstoff oder N' - [2 - (Dimethylamino)ethyl] - N - (1 - methylethyl) - N - [2 - [(2 - naphthyl)thio]ethyl]harnstoffsuccinat [1:1.5] oder N' - [2 - (Dimethylamino)ethyl] - N - (1 - methylethyl) - N - [2 - (1 - naphthylthio)ethyl]harnstoff oder N' - [2 - (Dimethylamino)ethyl] - N - (1 - methylethyl) - N - [2 - (1 - naphthylthio)ethyl]harnstoffoxalat.

4. Verfahren nach Anspruch 1, zur Herstellung von N - [2 - [(4 - Chlorphenyl)sulfonyl)ethyl] - N' - [2 - (dimethylamino)ethyl] - N - (1 - methylethyl)harnstoff oder N - [2 - [(4 - Chlorphenyl)sulfonyl) - ethyl] - N' - [2 - (dimethylamino)ethyl] - N - (1 - methylethyl)harnstoffmaleat [1:1] oder N - [2 - [(4 - Chlorphenyl)sulfonyl)ethyl] - N' - [2 - (diethylamino)ethyl] - N - (1 - methylethyl)harnstoff oder N - [2 - [(4 - Chlorphenyl)sulfonyl]ethyl] - N' - [2 - (diethylamino)ethyl] - N - (1 - methylethyl)harnstofftartrat [1:1] oder N - [2 - [(4 - Chlorphenyl)sulfonyl]ethyl] - N' - [2 - (dimethylamino)ethyl] - N' - (1 - methylethyl)harnstoff oder N - [2 - [(4 - Chlorphenyl)sulfonyl]ethyl] - N' - [2 - (dimethylamino)ethyl] - N' - (1 - methylethyl)harnstoffmaleat [1:1] oder N - [2 - [(4 - Chlorphenyl)sulfonyl]ethyl] - N' - [(dimethylaminoethyl] - N - (1 - methylethyl)thioharnstoff oder N - [2 - [(4 - Chlorphenyl)sulfonyl] - ethyl] - N' - [2 - (dimethylamino)ethyl] - N - (1 - methylethyl) - thioharnstoffhemifumarat.

5. Verfahren nach Anspruch 1, zur Herstellung von N' - [2 - (Dimethylamino)ethyl] - N - (1 - methylethyl) - N - [2 - (phenylsulfonyl) - ethyl]harnstoff oder N' - [2 - (Dimethylamino)ethyl] - N-(1 - methylethyl) - N - [2 - (phenylsulfonyl)ethyl]harnstoffmaleat [1:1] oder N' - [2 - (Diethylamino)ethyl] - N - (1 - methylethyl) - N - [2 - (phenylsulfonyl)ethyl]harnstoff oder N' - [2 - (Diethylamino)ethyl] - N - (1 - methylethyl) - N - [2 - (phenylsulfonyl)ethyl] - harnstoffmaleat [1:1] oder N' - [3 - (diethylamino)propyl] - N - (1 - methylethyl) - N - [3 - (phenylsulfonyl)propyl]harnstoff oder N' - [3 - (Diethylamino)propyl] - N - (1 - methylethyl) - N - [3 - (phenylsulfonyl)propyl]harnstoffoxalat - [1:1] - hemihydrat oder N' - [2 - (Diethylamino)ethyl] - N - (1 - methylethyl) - N - [3 - (phenylsulfonyl)propyl] - harnstoff oder N' - [2 - [(Diethylamino)ethyl] - N - (1 - methylethyl) - N - [3 - (phenylsulfonyl)propyl]-harnstoffoxalat - [1:1] - hemihyhydrat oder N' - [3 - (Diethylamino)propyl] - N - (1 - methylethyl) - N - [2 - (phenylsulfonyl)ethyl]harnstoff oder N' - [3 - (Diethylamino) - propyl] - N - (1 - methylethyl) - N - [2 - (phenylsulfonyl)ethyl] - harnstoff. 1/4 - Hydrat oder N' - [2 - (Diethylamino)ethyl] - N - [2 - [(4 - fluorphenyl)sulfonyl]ethyl] - N - (1-methylethyl)harnstoff.

6. Verfahren nach Anspruch 1, zur Herstellung von N' - [2 - (Dimethylamino)ethyl] - N - (1 - methylethyl) - N - [2 - [(1 - naphthyl) - sulfonyl]ethyl]harnstoff oder N' - [2 - (Dimethylamino)ethyl] - N - (1 - methylethyl) - N - [2 - [(1 - naphthyl)sulfonyl]ethyl] - harnstoffmaleat.

7. Verfahren nach Anspruch 1, zur Herstellung von N' - [2 - (Diethylamino)ethyl] - N - (1 - methylethyl) - N - [2 - [(4 - methylphenyl)sulfonyl]ethyl]harnstoff oder N' - [2 - (Diethylamino)ethyl] - N - (1 - methylethyl) - N - [2 - [(4 - methylphenyl)sulfonyl]ethyl] - harnstofftartrat oder N - [2 - [(3,4 - Dichlorphenyl)sulfonyl]ethyl] - N'(dimethylamino)ethyl] - N' - (1 - methylethyl)harnstoff oder N - [2 - [(3,4 - Dichlorphenyl)sulfonyl]ethyl] - N' - (dimethylamino)ethyl] - N - (1 - methylethyl)harnstoffmaleat.

8. Verfahren nach Anspruch 1, zur Herstellung von N - [2 - [(4 - Chlorphenyl)sulfinyl]ethyl] - N' - [2 - (dimethylamino)ethyl] - N - (1 - methylethyl)harnstoff oder N - [2 - [(4 - Chlorphenyl)sulfinyl] - ethyl] - N' - [2 - (dimethylamino)ethyl] - N - (1 - methylethyl)harnstoffumarat [1:1] oder N' - [2 - Diethylamino)-ethyl] - N - (1 - methylethyl) - N - [2 - [(4 - methylphenyl)sulfinyl]ethyl]]harnstoff oder N' - [2 - (Diethylamino)ethyl] - N - (1 - methylethyl) - N - [2 - [(4 - methylphenyl)sulfinyl]ethyl]harnstoffhemihydrat.

9. Verfahren zur Herstellung einer therapeutisch wirksamen Zusammensetzung für die Behandlung von Herzrhythmusstörungen, gekennzeichnet durch Mischen (a) einer wirksamen Menge einer

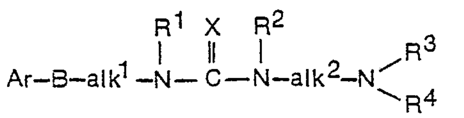

oder eines pharmazeutisch annehmbaren Additionssalzes oder Hydrates derselben und

(b) eines pharmazeutisch annehmbaren Trägers dafür.

10. Verfahren zur Herstellung einer therapeutisch wirksamen Zusammensetzung für die Behandlung von Herzrhythmusstörungen, gekennzeichnet durch Mischen

(a) einer wirksamen Menge einer der in einem der Ansprüche 2 bis 8 angeführten Verbindungen oder eines pharmazeutisch annehmbaren Additionssalzes oder Hydrates einer solche und

(b) eines pharmazeutisch annehmbaren Trägers dafür.